# EUROPEAN PATENT SPECIFICATION

(11) **EP 3 572 054 B1**
(45) Date of publication and mention of the grant of the patent: **17.02.2021**
(21) Application number: 17902546.5
(22) Date of filing: 21.03.2017
(51) Int. Cl.: A61F 13/15, G05B 19/418

(54) **METHOD AND DEVICE FOR MANUFACTURING ABSORBENT ARTICLE**
VERFAHREN UND VORRICHTUNG ZUR HERSTELLUNG SAUFÄHIGER ARTIKEL
PROCÉDÉ ET DISPOSITIF DE FABRICATION D'UN ARTICLE ABSORBANT

(43) Date of publication of application: 27.11.2019
(73) Proprietor: Unicharm Corporation, Shikokuchuo-shi, Ehime 799-0111 (JP)
(72) Inventor: HAGITA, Hiromi, Kanonji-shi Kagawa 769-1602 (JP); TASHIRO, Fumihiro, Kanonji-shi Kagawa 769-1602 (JP)
(74) Representative: Dolleymores
(86) International application number: PCT/JP2017/011209
(87) International publication number: WO 2018/173118

(56) References cited:
- EP-A1- 2 554 278
- WO-A1-2014/192983
- JP-A- 2006 012 132
- JP-A- 2006 065 598
- JP-A- 2011 200 833
- JP-B1- 6 089 155
- US-A1- 2004 083 018
- US-A1- 2004 083 018

## Description

### [Technical Field]

The present invention relates to a method and a device for manufacturing an absorbent article such as a sanitary napkin.

### [Background Art]

As an example of conventional absorbent articles, a sanitary napkin is known.

### [Citation List]

### [Patent Literature]

[PTL 1] Japanese Patent Application Publication No. 2010-234031

### [Summary of Invention]

### [Technical Problem]

The aforementioned napkin is manufactured in a manufacturing line. In the manufacturing line, a continuous sheet, which is continuous along a direction of transport, is transported in the direction of transport. At a predetermined position in the direction of transport, formation of a treated portion in each portion of the continuous sheet that is to become a napkin is performed in a repeated manner in the direction of transport. Specifically, a rotating body that rotates along the direction of transport is arranged at the aforementioned predetermined position in the direction of transport. Treatment processing sections are provided side-by-side along the rotation direction on the outer circumferential surface of the rotating body, and the treated portions are repeatedly formed by the treatment processing sections in the continuous sheet in the direction of transport.

Here, in the case where it is discovered that a napkin in the hands of a consumer has a defective treated portion, if it is possible to specify which of the treatment processing sections on the rotating body is the treatment processing section that formed the defective treated portion, the specified treatment processing section can be repaired immediately. This makes it possible to suppress the further production of defective products, therefore it is useful.

the present invention was achieved in light of conventional problems such as that described above, and an aspect of the present invention is to make it possible to easily specify which of a plurality of treatment processing sections on a rotating body is the treatment processing section that formed a certain treated portion.

### [Solution to Problem]

A main aspect of the present invention for achieving the above-described aspect is a method for manufacturing an absorbent article, including:
a transporting step of transporting a continuous sheet in a direction of transport,
   the continuous sheet being continuous in the direction of transport;
a treated-portion forming step of repeatedly forming a treated portion in a portion of the continuous sheet in the direction of transport,
   the portion being a unit portion that is to become the absorbent article,
   the treated-portion forming step being performed at a predetermined position in the direction of transport,
   the treated-portion forming step including forming the treated portions by a plurality of treatment processing sections,
      the plurality of treatment processing sections being provided at different positions in a rotation direction on an outer circumferential surface of a rotating body,
      the rotating body being arranged at the predetermined position and rotating along the direction of transport; and
an information generating step of generating information regarding either of each treated portion and the absorbent article that has that treated portion,
the information generating step including generating, as the information, correlation information for correlating either of each treated portion and each absorbent article having that treated portion with the treatment processing section which has formed that treated portion among the plurality of treatment processing sections.

Further, a device for manufacturing an absorbent article, including:
a transporting device configured to transport a continuous sheet in a direction of transport,
   the continuous sheet being continuous in the direction of transport;
a treated-portion forming device configured to repeatedly form a treated portion in a portion of the continuous sheet in the direction of transport,
   the portion being a unit portion that is to become the absorbent article,
   the forming being performed at a predetermined position in the direction of transport,
   the treated-portion forming device forming the treated portions by a plurality of treatment processing sections,
      the plurality of treatment processing sections being provided at different positions in a rotation direction on an outer circumferential surface of a rotating body,
      the rotating body being arranged at the predetermined position in the direction of transport and rotating along the direction of transport; and
an information generation device configured to generate information regarding either of each treated portion and the absorbent article that has that treated portion,
   the information generation device generating, as the information, correlation information for correlating either of each treated portion and each absorbent article having that treated portion with the treatment processing section which has formed that treated portion among the plurality of treatment processing sections.

Features of the present invention other than the above will become clear by reading the description of the present specification with reference to the accompanying drawings.

### [Advantageous Effects of Invention]

According to the present invention, it is possible to easily specify which of a plurality of treatment processing sections on a rotating body is the treatment processing section that formed a certain treated portion.

### [Brief Description of the Drawings]

FIG. 1A is a schematic plan view of a sanitary napkin 1 that is one example of an absorbent article, and FIG. 1B is a cross sectional view taken along a line B-B indicated by an arrows.
FIG. 2 is a schematic side view of a manufacturing line Lm for manufacturing the napkin 1.
FIG. 3 is an illustrative view of a recording area.
FIG. 4 is an illustrative view of a recording area obtained by further providing a protruding-portion-number cell in the recording area in FIG. 3.
FIG. 5A is a diagram showing a correct correspondence between protruding-portion ID numbers and recessed-portion ID numbers in an embodiment.
FIG. 5B is a diagram showing a correct correspondence between protruding-portion ID numbers and recessed-portion ID numbers in a comparative example.
FIG. 6 is a diagram showing a correct correspondence between protruding-portion ID numbers and recessed-portion ID numbers in the case where, as a more preferable example, the number of provided protruding portions 31p is the prime number "2", and the number of provided recessed portions 11h is "3", which is not an integral multiple of the number of provided protruding portions 31p.

### [Description of Embodiments]

At least the following matters will become clear with the description of this specification and the attached drawings.

A method for manufacturing an absorbent article, including:
a transporting step of transporting a continuous sheet in a direction of transport,
   the continuous sheet being continuous in the direction of transport;
a treated-portion forming step of repeatedly forming a treated portion in a portion of the continuous sheet in the direction of transport,
   the portion being a unit portion that is to become the absorbent article,
   the treated-portion forming step being performed at a predetermined position in the direction of transport,
   the treated-portion forming step including forming the treated portions by a plurality of treatment processing sections,
      the plurality of treatment processing sections being provided at different positions in a rotation direction on an outer circumferential surface of a rotating body,
      the rotating body being arranged at the predetermined position and rotating along the direction of transport; and
an information generating step of generating information regarding either of each treated portion and the absorbent article that has that treated portion,
   the information generating step including generating, as the information, correlation information for correlating either of each treated portion and each absorbent article having that treated portion with the treatment processing section which has formed that treated portion among the plurality of treatment processing sections.

According to this method for manufacturing an absorbent article, information is generated which is for correlating each treated portion or absorbent article having that treated portion with the treatment processing section which has formed that treated portion among the plurality of treatment processing sections. Accordingly, it is possible to, at a later time, easily specify which of the treatment processing sections on the rotating body is the treatment processing section that formed a certain treated portion.

In such a method for manufacturing an absorbent article, it is desirable that
letting the treated portions be first treated portions, the rotating body be a first rotating body, the predetermined position be a first predetermined position, and the treatment processing sections be first treatment processing sections,
the first treatment processing sections are provided at a first angular interval in the rotation direction,
the method further comprises a second treated-portion forming step of forming a second treated portion in each first treated portion,
the second treated-portion forming step is performed at a second predetermined position in the direction of transport that is different from the first predetermined position,
the second treated-portion forming step includes forming the second treated portions by several second treatment processing sections,
the several second treatment processing sections are provided at a second angular interval in a rotation direction on an outer circumferential surface of a second rotating body,
the second rotating body being arranged at the second predetermined position and rotating along the direction of transport,
the correlation information is information for correlating a first treatment processing section with a second treatment processing section,
   the first treatment processing section being one of the plurality of first treatment processing sections and forming a certain first treated portion,
   the second treatment processing section being one of the several second treatment processing sections and forming the second treated portion in the certain first treated portion, and
the number of first treatment processing sections provided on the first rotating body is different from the number of second treatment processing sections provided on the second rotating body.

According to this method for manufacturing an absorbent article, the number of provided first treatment processing sections is different from the number of provided second treatment processing sections. Accordingly, by comparing the above-described information with absorbent-article defective-product information and examining the defective product production pattern, it is possible to easily determine whether a first treatment processing section or a second treatment processing section is the cause of the defect.

In such a method for manufacturing an absorbent article, it is desirable
that either of the number of first treatment processing sections provided on the first rotating body and the number of second treatment processing sections provided on the second rotating body is a prime number, and
that the other one of the numbers is not an integral multiple (excluding 0 and 1) of the prime number.

According to this method for manufacturing an absorbent article, either of the number of provided first treatment processing sections and the number of provided second treatment processing sections is a prime number, and the other one of the numbers is not an integral multiple of the prime number. Accordingly, by comparing the above-described information with absorbent-article defective-product information and examining the defective product production pattern, it is possible to more easily determine whether a first treatment processing section or a second treatment processing section is the cause of the defect.

In such a method for manufacturing an absorbent article, it is desirable
that rotation of the rotating body and transporting of the continuous sheet are coordinated with each other based on a synchronization signal constituted by a unit signal that is repeatedly output,
that each time the unit signal is output, only one of the portions that are to become the absorbent articles in the continuous sheet is transported in the direction of transport,
that a treatment-processing-section identifier is non-redundantly set for each treatment processing section,
that each time the unit signal is output,
the rotating body rotates such that a treated portion is formed by one of the treatment processing sections, and
information indicating the treatment-processing-section identifier of the one treatment processing section which has formed that treated portion is output as the correlation information,
that at a third predetermined position in the direction of transport, each time the unit signal is output,
the absorbent article or a portion to become the absorbent article passes the third predetermined position,
an identifier is non-redundantly provided on the passing absorbent article or portion to become the absorbent article, and
information indicating the identifier is output as the correlation information, and
that the information indicating the identifier and the information indicating the treatment-processing-section identifier are correlated with each other based on a sum of a number of absorbent articles and a number of portions to become the absorbent article,
the absorbent articles and the portions being those existing between the predetermined position and the third predetermined position.

According to this method for manufacturing an absorbent article, the information indicating the identifier is correlated with the treatment-processing-section identifier information based on the number in total of absorbent articles and portions to become absorbent articles, which are to exist between the predetermined position and the third predetermined position. Accordingly, it is possible to reliably correlate each treated portion or absorbent article having the treated portion with the treatment processing section that formed that treated portion among the plurality of treatment processing sections.

In such a method for manufacturing an absorbent article, it is desirable that
among the absorbent articles, an absorbent article determined to be a defective product is discharged at a position downstream in the direction of transport with respect to the third predetermined position.

According to this method for manufacturing an absorbent article, a discharged absorbent article that was determined to be a defective product is also provided with an identifier. Accordingly, before a defective absorbent article reaches the consumer, the treatment processing section that formed the treated portion of the defective absorbent article can be specified based on the treatment-processing-section identifier information that corresponds to the identifier information of the defective absorbent article. Accordingly, it is possible to relatively quickly inspect and repair that treatment processing section, and as a result, it is possible to effectively prevent the further production of defective products.

In such a method for manufacturing an absorbent article, it is desirable that
the method further comprises:
a step of placing a plurality of the absorbent articles into one packaging bag body; and a second information generating step of generating second information,
   the second information being information for correlating the plurality of absorbent articles with the packaging bag body into which the absorbent articles were placed.

According to this method for manufacturing an absorbent article, the second information is generated in order to correlate the plurality of absorbent articles with the packaging bag body into which the absorbent articles were placed. Accordingly, if a defective absorbent article is discovered at a later time, the packaging bag body that includes that defective absorbent article can be swiftly specified based on the second information. By collecting absorbent articles together with one packaging bag body at a time, it is possible to increase efficiency in the collection of defective absorbent articles compared with the case of collecting defective absorbent articles individually.

Further, a device for manufacturing an absorbent article, including:
a transporting device configured to transport a continuous sheet in a direction of transport,
   the continuous sheet being continuous in the direction of transport;
a treated-portion forming device configured to repeatedly form a treated portion in a portion of the continuous sheet in the direction of transport,
   the portion being a unit portion that is to become the absorbent article,
   the forming being performed at a predetermined position in the direction of transport,
   the treated-portion forming device forming the treated portions by a plurality of treatment processing sections,
      the plurality of treatment processing sections being provided at different positions in a rotation direction on an outer circumferential surface of a rotating body,
      the rotating body being arranged at the predetermined position in the direction of transport and rotating along the direction of transport; and
an information generation device configured to generate information regarding either of each treated portion and the absorbent article that has that treated portion,
   the information generation device generating, as the information, correlation information for correlating either of each treated portion and each absorbent article having that treated portion with the treatment processing section which has formed that treated portion among the plurality of treatment processing sections.

With such a device for manufacturing an absorbent article, it is possible to achieve actions and effects similar to those in the case of the manufacturing method described above.

### Embodiment

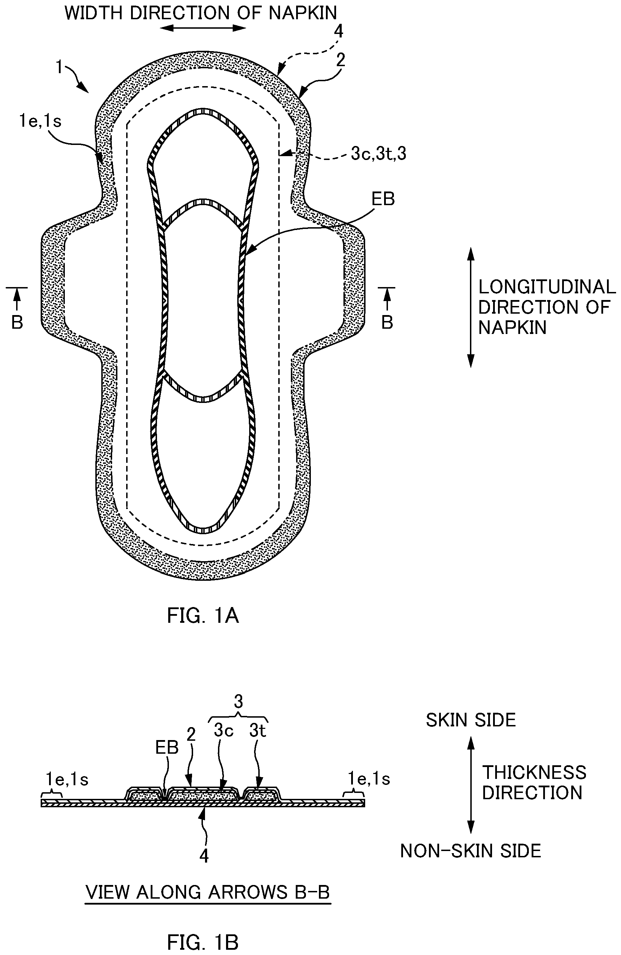

A method and a device for manufacturing an absorbent article 1 of an embodiment of the present invention are used in a manufacturing line Lm for a sanitary napkin 1 that is one example of the absorbent article 1. FIG. 1A is a schematic plan view of the napkin 1, and FIG. 1B is a cross sectional view taken along a line B-B indicated by an arrows.

In an external view, the napkin 1 has an approximately planar shape having a longitudinal direction, a width direction, and a thickness direction. The napkin 1 includes: a liquid-impermeable back sheet 4 made of a resin film or the like; a liquid-permeable top sheet 2 made of a nonwoven fabric sheet or the like; and a liquid-absorbent absorbent body 3 that is arranged between these sheets 2 and 4 in the thickness direction. The top sheet 2 and the back sheet 4 are joined by seal portions 1s that are formed at outer edge portions 1e of the planar shape set in the longitudinal direction and the width direction, and therefore the absorbent body 3 is inseparably held between the two sheets 2 and 4.

Note that in this example, the portion of the top sheet 2 that covers the absorbent body 3 is provided with an approximately loop-shaped emboss groove EB that is formed by pressing. Thus, the top sheet 2 and the absorbent body 3 are integrally joined by being pressed together. Also, the absorbent body 3 has an absorbent core 3c and tissue paper 3t that covers the outer surface of the core 3c. The absorbent core 3c is obtained by molding a liquid absorbent material made of pulp fibers, a superabsorbent polymer (so-called SAP), or the like into a predetermined shape. Furthermore, a production number (not shown) is printed on a portion of the back sheet 4 as an identifier for being distinguished from another napkin 1.

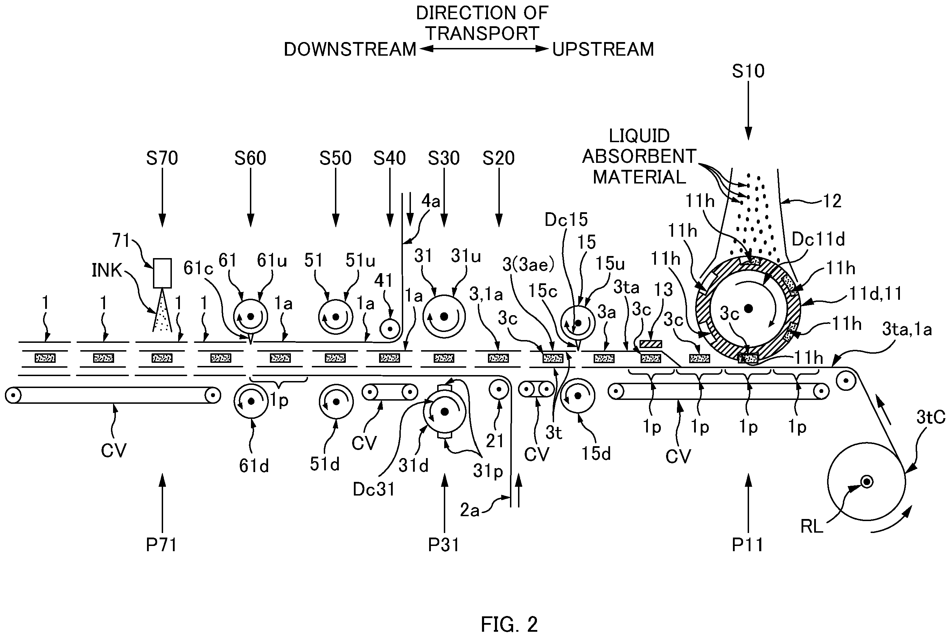

FIG. 2 is a schematic side view of the manufacturing line Lm for manufacturing the napkin 1.

In this manufacturing line Lm, semi-finished goods 1a of the napkin 1 are continuously transported along a predetermined direction of transport by appropriate transporting mechanisms CV, CV... or the like. During this transporting, the various portions of the semi-finished goods 1a are subjected to processing such as joining, pressing, and punching, the state of the semi-finished goods 1a successively changes in each process, and the napkin 1 in FIG. 1A is ultimately produced.

It should be noted that in this example, the semi-finished goods 1a are basically transported in a so-called logitudinal-direction flow manner. Specifically, the semi-finished goods 1a are transported in a state where the direction corresponding to the longitudinal direction of the napkin 1 (FIG. 1A) is aligned with the direction of transport.

Also, the transporting mechanisms CV that transport the semi-finished goods 1a include: a suction belt conveyor having a function for suctioning and holding a target to a belt surface (the placement surface); and a belt conveyor or a transporting roller that forms a transporting route for the semi-finished goods 1a between a pair of endless belts arranged on upper and lower sides, for example. Note that in this example, all of the transporting mechanisms CV use a servomotor as a drive source, but there is no limitation whatsoever to this.

Along the transporting route for the semi-finished goods 1a, the manufacturing line Lm has an absorbent-body forming step S10, a top-sheet supplying step S20, an emboss-groove treatment step S30, a back-sheet supplying step S40, a round-seal treatment step S50, a dividing step S60, and a production-number printing step S70.

The following describes these steps S10 to S70, and in this description, out of the two directions that are orthogonal to the direction of transport of the semi-finished goods 1a, the direction that is not the thickness direction of the semi-finished goods 1a (i.e., the direction that passes through the paper plane in FIG. 2) will be referred to as the "CD direction".

In the first absorbent-body forming step S10, the absorbent body 3 is formed. First, a continuous sheet 3ta of the tissue paper 3t (corresponding to a "continuous sheet", which will be simply called the tissue paper 3ta hereinafter) is fed from a material coil 3tC of the tissue paper 3t that is rotatably supported on an appropriate feeding device RL. And the tissue paper 3ta is continuously transported (corresponding to a transporting step) in the direction of transport by a transporting device CV (corresponding to a transporting device). A rotating drum device 11 is arranged at a predetermined position P11 in the direction of transport (corresponding to a first predetermined position). While passing the position of rotating drum device 11, the absorbent core 3c (one example of a treated portion) is placed on the upper surface of the tissue paper 3ta (corresponding to a treated-portion forming step). The absorbent cores 3c, 3c... are transported downstream in the direction of transport side-by-side intermittently on the tissue paper 3ta, or in other words, with appropriate gaps between pairs of absorbent cores 3c that are adjacent in the direction of transport. Downstream thereof, there is a folding guide member 13 which is constituted by an appropriate plate, roller, or the like. Then, while passing the position of the folding guide member 13, the tissue paper 3ta is folded by the folding guide member 13 into a tubular shape having a tube axis that extends in the direction of transport. Thus, the absorbent core 3c is covered by the tube-shaped tissue paper 3ta. Accordingly, a continuous body 3a of the absorbent body 3 is produced. Then, when the continuous body 3a passes the position of an absorbent-body cutter device 15 on the downstream side, the downstream end portion 3ae of the continuous body 3a is cut such that one absorbent core 3c is included in that end portion 3ae, thus obtaining a cut-form absorbent body 3.

The rotating drum device 11 (corresponding to a treated-portion forming device) and the absorbent-body cutter device 15 will be described in more detail below. Firstly, the rotating drum device 11 includes: a rotating drum 11d (corresponding to a rotating body and a first rotating body) that is supported so as to be capable of rotating about a rotation axis that extends in the CD direction; and a servomotor (not shown) that serves as a drive source for driving the rotating drum 11d to rotate. The drum 11d is driven to rotate along the direction of transport in coordination with the transporting of the tissue paper 3ta.

Also, a plurality of recessed portions 11h, 11h... (corresponding to treatment processing sections and first treatment processing sections) are provided on the outer circumferential surface of the rotating drum 11d at different positions from each other in a rotation direction Dc11d in order to suction and form a layer of a liquid absorbent material such as pulp fibers. More specifically, as one example, six recessed portions 11h, 11h... are provided at an angular interval of 60°, which is one example of a first angular interval. Also, a duct 12 for supplying the liquid absorbent material is arranged above the rotating drum 11d. Accordingly, when each of the recessed portions 11h of the rotating drum 11d passes the position of the duct 12, the liquid absorbent material is suctioned by the recessed portion 11h and forms a layer therein, thus forming one absorbent core 3c. Thereafter, when, in the rotation direction Dc11d of the rotating drum 11d, the recessed portion 11h passes the position where the tissue paper 3ta is transported, the absorbent core 3c is passed from the recessed portion 11h and placed on the upper surface of the tissue paper 3ta.

Also, the absorbent-body cutter device 15 includes, for example, a pair of upper and lower rolls 15u and 15d and a servomotor (not shown) . The pair of upper and lower rolls 15u and 15d are supported so as to be capable of rotating about a rotation axis that extends in the CD direction. The servomotor serves as a drive source for driving the rolls 15u and 15d to rotate. The upper roll 15u is a cutter roll 15u that has a cutter blade 15c on the outer circumferential surface, and the lower roll 15d is an anvil roll 15d that receives the cutter blade 15c. In this example, only one cutter blade 15c is provided on the outer circumferential surface of the cutter roll 15u. Accordingly, one absorbent body 3 is produced by being cut from the continuous body 3a of the absorbent body 3 each time the upper roll 15u makes one full rotation. The produced absorbent body 3 is subsequently sent to a transporting mechanism CV on the downstream side.

In the next top-sheet supplying step S20, a continuous sheet 2a of the top sheet 2 (hereinafter, also simply called the top sheet 2a) is continuously supplied toward the absorbent bodies 3, 3..., which are the semi-finished goods 1a at that time, by a lower supply roll 21. Accordingly, the undersides of the absorbent bodies 3, 3... are covered by the top sheet 2a.

In the next emboss-groove treatment step S30, an emboss-groove treatment device 31 performs compression to form, from below, the emboss groove EB (FIG. 1A) in the lower surface of the top sheet 2a, which is the surface that corresponds to the skin-side surface, and the top sheet 2a and the absorbent body 3 are thus integrally joined together.

This emboss-groove treatment device 31 is provided at a predetermined position P31 (corresponding to a second predetermined position) in the direction of transport, and includes a pair of upper and lower rolls 31u and 31d and a servomotor (not shown). The pair of upper and lower rolls 31u and 31d have outer circumferential surfaces that face each other and are supported so as to be capable of rotating about a rotation axis that extends in the CD direction. The servomotor serves as a drive source for driving the rolls 31u and 31d to rotate.

The lower roll 31d has protruding portions 31p on the outer circumferential surface, whereas the upper roll 31u has a smooth outer circumferential surface in order to receive the protruding portions 31p. The protruding portions 31p project from the outer circumferential surface of the lower roll 31d and have a shape that corresponds to the formation pattern of the emboss groove EB. Accordingly, when the absorbent body 3 passes through the roll gap between the rolls 31u and 31d, the absorbent body 3 is clamped together with the top sheet 2a by the protruding portion 31p and the outer circumferential surface of the upper roll 31u at a predetermined pressing load. The emboss groove EB is thus formed, by pressing, in the portion of the top sheet 2a that covers the absorbent body 3.

Note that in this example, a plurality of the protruding portions 31p are provided on the outer circumferential surface of the lower roll 31d at different positions from each other in the rotation direction Dc31. More specifically, in this example, two protruding portions 31p are provided at an angular interval of 180°, which is one example of a second angular interval, in the rotation direction Dc31. Accordingly, each time an absorbent body 3 passes, the lower roll 31d rotates one-half rotation, and one emboss groove EB is thus formed in each absorbent body 3 through the top sheet 2a.

In the next back-sheet supplying step S40, a continuous sheet 4a of the back sheet 4 (hereinafter also simply called the back sheet 4a) is supplied to the semi-finished goods 1a by a supply roll 41 that is located on the upward side. The back sheet 4a is arranged so as to sandwich the absorbent body 3 along with the top sheet 2a, and the portions of the back sheet 4a that protrude laterally from the absorbent body 3 are overlaid on the top sheet 2a. Accordingly, in the semi-finished goods 1a, the absorbent body 3 is arranged between the top sheet 2a and the back sheet 4a.

In the next round-seal treatment step S50, seal portions 1s that correspond to outer edge portions 1e (FIG. 1A) of the napkin 1 are formed in the semi-finished goods 1a, and thus the sheets 2a and 4a are welded and joined to each other in the portions corresponding to the outer edge portions 1e. The processing for forming the seal portions 1s is performed by a round-seal treatment device 51.

The round-seal treatment device 51 includes a pair of upper and lower rolls 51u and 51d and a servomotor (not shown). The pair of upper and lower rolls 51u and 51d have outer circumferential surfaces that face each other and are supported so as to be capable of rotating about a rotation axis that extends in the CD direction. The servomotor serves as a drive source for driving the rolls 51u and 51d to rotate.

The lower roll 51d has a protruding portion (not shown) on the outer circumferential surface, and the upper roll 51u has a smooth outer circumferential surface in order to receive the protruding portion. The protruding portion projects from the outer circumferential surface of the lower roll 51d and has a shape that corresponds to the outer edge portions 1e of the napkin 1. Accordingly, when the semi-finished goods 1a pass through the roll gap between the upper and lower rolls 51u and 51d, the portions of the semi-finished goods 1a that correspond to the outer edge portions 1e of the napkin 1 are clamped by the protruding portion and the outer circumferential surface of the upper roll 51u at a predetermined pressing load. Thus, seal portions 1s are formed, by pressing, in the portions of the semi-finished goods 1a that correspond to the outer edge portions 1e of the napkin 1.

Note that in this example, only one protruding portion is provided on the outer circumferential surface of the lower roll 51d. Accordingly, each time one portion 1p that is to become the napkin 1 passes, the lower roll 51d rotates one full rotation, and one seal portion 1s is thus formed for each portion that is to become the napkin 1.

In the next dividing step S60, the semi-finished goods 1a that are continuous in the direction of transport are cut into individual products, thus producing cutform napkins 1. This division processing is performed by a die cutter device 61.

The die cutter device 61 includes a pair of upper and lower rolls 61u and 61d and a servomotor (not shown). The pair of upper and lower rolls 61u and 61d have outer circumferential surfaces that face each other and are supported so as to be capable of rotating about a rotation axis that extends in the CD direction. The servomotor serves as a drive source for driving the rolls 61u and 61d to rotate. The upper roll 61u is a cutter roll 61u that has a cutter blade 61c on the outer circumferential surface, and the lower roll 61d is an anvil roll 61d that has a smooth outer circumferential surface for receiving the cutter blade 61c. The cutter blade 61c projects from the outer circumferential surface of the cutter roll 61u with a shape that corresponds to the above-described outer shape of the napkin 1. Accordingly, when the semi-finished goods 1a pass through the roll gap between the upper and lower rolls 61u and 61d, a napkin 1 is produced by being punched out from the semi-finished goods 1a.

Note that in this example, only one cutter blade 61c is provided on the upper roll 61u. Accordingly, only one napkin 1 is produced by being punched out from the semi-finished goods 1a each time the upper roll 61u rotates one full rotation. The punched-out napkin 1 is then sent downstream in the direction of transport.

In the next production-number printing step S70, a production number is non-redundantly printed on each of the napkins 1 that have been produced by being punched out. This printing is performed by an inkjet printer 71, which is one example of a printing device. Specifically, this printer 71 includes nozzles (not shown) and piezoelectric elements (not shown) as drive sources for ejecting ink from the nozzles. Successive numbers are printed as production numbers by such ejection of ink, and this printing is performed on all of the napkins 1 that pass a direction-of-transport position P71 at which the printer 71 is provided (ink ejection processing position P71). Note that these production numbers are used for investigating the cause in the manufacturing line Lm when a defective absorbent core 3c is discovered in a napkin 1 in the hands of a consumer, for example. This will be described later.

It should be noted that the operations in the steps S10 to S70 in the manufacturing line Lm are performed in coordination with each other. In other words, the devices 11, 15, 31, 51, 61, and 71 used in at least the steps S10, S30, S50, S60, and S70 operate in synchronization with each other based on a synchronization signal.

Here, this synchronization signal includes unit signals that are repeatedly output for each napkin 1, and in this example, a rotation angle signal that is indicated by a rotation angle value from 0° to 360° is output as each unit signal. The devices 11, 15, 31, 51, 61, and 71 have respectively unit processing operations, each of which has undergone patterning in order to be repeated for each napkin 1 or each portion 1p that is to become a napkin. These unit processing operations are correlated with the unit signals in one-to-one correspondence.

Accordingly, the synchronization signal is transmitted to the drive sources (e.g., servomotors) of the devices 11, 15, 31, 51, 61, and 71 for the steps S10, S30, S50, S60, and S70. The positions of the drive sources are controlled based on the synchronization signal, and therefore the devices 11, 15, 31, ..., and 71 perform predetermined unit processing operations on the napkin 1 or on the portion 1p that is to become the napkin 1 in the semi-finished goods 1a. As a result, the devices 11, 15, 31, ..., and 71 perform corresponding treatment for each napkin 1 or for each portion 1p that is to become a napkin 1 in the semi-finished goods 1a at the corresponding direction-of-transport positions where the devices 11, 15, 31, ..., and 71 are arranged.

For example, the rotating drum 11d of the rotating drum device 11 for the absorbent-body forming step S10 has six recessed portions 11h for forming the absorbent core 3c, and the six recessed portions 11h are arranged at positions along the rotation direction Dc11d. Accordingly, the rotating drum 11d is controlled so as to rotate by one-sixth of a full rotation (rotate 60°) as a unit processing operation each time the unit signal of the synchronization signal is output. Also, the speed of the servomotor of a transporting mechanism CV in the vicinity of the rotating drum 11d is also controlled based on the synchronization signal. And thus, concerning the tissue paper 3ta which is the semi-finished goods 1a at this direction-of-transport position, only one of the portions 1p (to become the napkins 1) of the tissue paper 3ta is transported each time the unit signal of the synchronization signal is output. Accordingly, one absorbent core 3c is formed for each portion 1p that is to become the napkin 1 in the tissue paper 3ta.

Also, on the upper roll 15u of the absorbent-body cutter device 15 in the step S10, only one cutter blade 15c is provided in the rotation direction Dc15. Accordingly, each time the unit signal of the synchronization signal is output, the positions of the upper and lower rolls 15u and 15d are controlled so as to make one full rotation as the unit processing operation. Also, the speed of the servomotor of a transporting mechanism CV in the vicinity of the upper and lower rolls 15u and 15d is also controlled based on the synchronization signal. And thus, concerning the continuous body 3a of the absorbent body 3 which is the semi-finished goods 1a at this direction-of-transport position, only one portion 1p (to become the napkin 1) of the continuous body 3a is transported each time the unit signal of the synchronization signal is output. Accordingly, one absorbent body 3 is cut away for each portion 1p that is to become the napkin 1 in the continuous body 3a of the absorbent body 3.

Note that the emboss-groove treatment device 31 in the emboss-groove treatment step S30, the round-seal treatment device 51 in the round-seal treatment step S50, and the die cutter device 61 in the dividing step S60 all perform corresponding processing by means of a similar technique based on the synchronization signal, and all can be easily envisioned based on the above descriptions. In other words, based on the fact that the positions of the devices 31, 51, and 61 are controlled based on the synchronization signal, and the fact that the speeds of the transporting mechanisms CV in the vicinities thereof are controlled based on the synchronization signal, it can be easily understood from the above description that the devices 31, 51, and 61 perform corresponding processing for each portion 1p that is to become a napkin 1 in the semi-finished goods 1a at the direction-of-transport positions where the devices 31, 51, and 61 are respectively provided. A further description therefore will not be given for this.

The printer 71 in the production-number printing step S70 has nozzles that eject ink based on the synchronization signal. Accordingly, each time the unit signal of the synchronization signal is output, ink is ejected from the nozzles so as to print a production number on the napkin 1. Also, the speed of the servomotor of a transporting mechanism CV in the vicinity of the printer 71 is also controlled based on the synchronization signal. And thus, only one napkin 1 (a product) is transported each time the unit signal of the synchronization signal is output. Accordingly, one production number is printed for each napkin 1.

This synchronization signal is generated by a controller that controls the devices 11, 15... of the manufacturing line Lm, for example. Specifically, the controller is a so-called computer or programmable logic controller (PLC), and has a memory that stores a program for generating the synchronization signal. A processor of the controller reads out the program from the memory and executes the program so as to repeatedly generate the unit signals of the synchronization signal, and transmits the synchronization signal to the drive sources of the devices 11, 15, and so on.

Note that the unit signals of the synchronization signal are signals indicated by a rotation angle value from 0° to 360° in order to facilitate understanding in this example, but there is no limitation whatsoever to this. For example, the unit signals of the synchronization signal may be indicated by a digital value from 0 to 8191.

In addition to the program for generating the synchronization signal, the memory of the controller also stores programs involved in various types of processing, such as programs for controlling the devices 11, 15... of the manufacturing line Lm. For example, a program for performing later-described processing, that is to say a program for generating recessed-portion ID number information and production number information and recording such information in a recording area of the memory, and a program for correlating such pieces of information with each other are also stored in advance in the memory. By reading out such programs from the memory and executing them, the controller (corresponding to an information generation device) performs processing for generating such information and correlating the pieces of information with each other.

As mentioned in the above description of conventional problems, assuming the case where it is discovered that a napkin 1 in the hands of a consumer is a defective product, such as the case where it is discovered that the napkin 1 has a defect in the absorbent core 3c of the absorbent body 3. In that case, if it is possible to specify which of the six recessed portions 11h, 11h... of the rotating drum 11d of the rotating drum device 11 is the recessed portion 11h that formed the defective absorbent core 3c, this makes it possible to immediately repair that recessed portion 11h and therefore it is useful.

In view of this, in the present embodiment, an innovation has been made to make this possible. Specifically, information is generated in order to correlate each napkin 1 having an absorbent core 3c with the recessed portion 11h that formed that absorbent core 3c out of the six recessed portions 11h, 11h... of the rotating drum 11d. This will be described in detail below.

First, non-redundant recessed-portion ID numbers are set for the recessed portions 11h of the rotating drum 11d as identifiers (corresponding to treatment-processing-section identifiers) for being distinguished from other recessed portions 11h. Because six recessed portions 11h, 11h... are provided in this example, the six consecutive numbers from "1" to "6" are set as the recessed-portion ID numbers in order from downstream to upstream in the rotation direction Dc11d. Note that the term "set" used here means providing a corresponding recessed-portion ID number for each recessed portion 11h in a visually recognizable manner for example, and it is possible to engrave or ink-print the corresponding recessed-portion ID number in the vicinity of each recessed portion 11h of the rotating drum 11d, for example.

Also, as previously mentioned, one absorbent core 3c is placed on the tissue paper 3ta each time a unit signal of the synchronization signal is output. Here, each time the unit signal is output, concerning the recessed portion 11h that is placing the absorbent core 3c on the tissue paper 3ta at the time of the outputting, the previously mentioned controller generates and outputs information indicating the recessed-portion ID number thereof (corresponding to an information generating step). For example, when the unit signal is output, if the recessed portion 11h having the recessed-portion ID number "1" is placing the absorbent core 3c on the tissue paper 3ta, the controller outputs "1" as the recessed-portion ID number information. Then when the recessed portion 11h having the recessed-portion ID number "2" is placing the absorbent core 3c on the tissue paper 3ta, the controller outputs "2" as the recessed-portion ID number information.

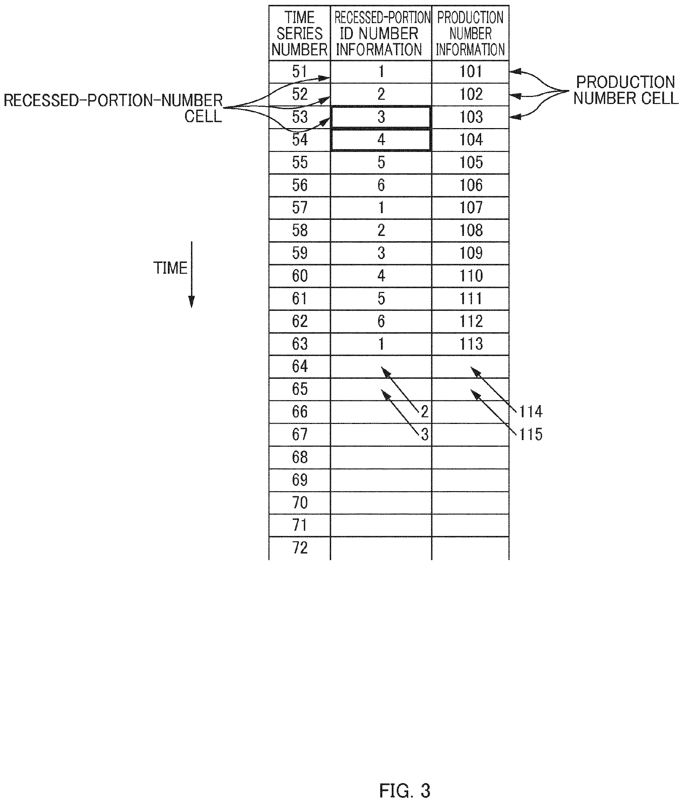

The memory of the controller also has a recording area for recording the aforementioned recessed-portion ID number information in the order in which such information is output. FIG. 3 is an illustrative view of the recording area. This area has recessed-portion-number cells that each record one piece of the aforementioned information, and the recessed-portion-number cells are assigned numbers that indicate a time series (hereinafter, also called time series numbers), such as consecutive numbers beginning with "1", in time series order. Each time a piece of recessed-portion ID number information is output, that information is recorded in the recessed-portion-number cell that has the smallest time series number among the empty recessed-portion-number cells. For example, in the example in FIG. 3, at the time when information indicating the recessed-portion ID number "2" is output, the time series number "64" is the smallest among the empty recessed-portion-number cells, and therefore "2" is recorded as the recessed-portion ID number information in the cell that corresponds to the time series number "64", and then when information indicating the recessed-portion ID number "3" is output, the time series number "65" is the smallest among the empty recessed-portion-number cells, and therefore "3" is recorded as the recessed-portion ID number information in the cell that corresponds to the time series number "65".

Also, a previously mentioned, the printer 71 also prints production numbers on the napkins 1 when the unit signals of the synchronization signal are output. In this print processing, the previously mentioned controller generates production number information (corresponding to an information generating step and a second information generating step), and outputs such information to the printer 71 in real-time. Also, each time a unit signal is output, information indicating the production number that is being printed on the napkin 1 when that unit signal is output is also recorded in real-time in the recording area in the time series.

Accordingly, a piece of recessed-portion ID number information and a piece of production number information that correspond to processing performed at the time of the output of the same unit signal are recorded in association with the same time series number.

For example, as shown in FIG. 3, the recording area is provided with production number cells for recording production number information in association with the recessed-portion-number cells. When a unit signal is output in order to cause the recessed portion 11h having the recessed-portion ID number "2" to place the absorbent core 3c on the tissue paper, if the production number "114" is being printed, the controller records "114" as the production number information in the production number cell that corresponds to the time series number "64". Then when a unit signal is output in order to cause the recessed portion 11h having the recessed-portion ID number "3" to place the absorbent core 3c on the tissue paper, if the production number "115" is being printed, the controller records "115" as the production number information in the production number cell that corresponds to the time series number "65". Accordingly, a piece of recessed-portion ID number information and a piece of production number information that correspond to processing performed at the time of the output of the same unit signal are recorded in association with the same time series number.

It should be noted that, as can be understood from FIG. 2, the predetermined position P11 in the direction of transport for forming the absorbent core 3c and the predetermined position P71 (corresponding to a third predetermined position) in the direction of transport for printing the production number are shifted from each other in the direction of transport by 11 in total of napkins 1 and portions 1p that are to become napkins 1.

For this reason, a piece of production number information and a piece of recessed-portion ID number information that are correlated with the same time series number in the recording area do not indicate the same napkin 1 as each other, that is to say, the correspondence between the production number information and the recessed-portion ID number information recorded in the recording area is shifted from the correct correspondence, more specifically is shifted by 11 in total of napkins 1 and portions 1p, which exist between the two predetermined positions P11 and P71.

In view of this, in order to find the correct correspondence, the controller shifts the correspondence between the production number information and the recessed-portion ID number information in the recording area by 11 places in the time series.

For example, in the case of the information recorded in the recording area shown in FIG. 3, for the production number information "114", the recessed-portion ID number that is 11 places in the past in the time series, namely "3", is acquired as the correct number information, and for the next production number information "115", the recessed-portion ID number that is 11 places in the past in the time series, namely "4", is acquired as the correct number information. Accordingly, it is possible to correctly recognize correlating with each other: the piece of information indicating the production number that was printed on a napkin 1; and the piece of information indicating the recessed-portion ID number of the recessed portion 11h that formed the absorbent core 3c of the napkin 1 that has that production number. As a result, it is possible to specify which of the six recessed portions 11h, 11h... of the rotating drum 11d is the recessed portion 11h that formed the defective absorbent core 3c.

Also, in the emboss-groove treatment step S30, the previously described emboss groove EB is formed in not only the top sheet 2a but also the absorbent body 3. At that time, since the absorbent core 3c makes up the main body of the absorbent body 3, the emboss groove EB (corresponding to a second treated portion) is substantially formed in the core 3c (corresponding to a first treated portion) as well. Accordingly, if the napkin 1 in the hands of a consumer has a defective absorbent core 3c as described above, there is also a possibility that the defect was caused by the emboss-groove treatment device 31 that formed the emboss groove EB.

For this reason, it is desirable that the innovation made for the rotating drum device 11 is made not only for the rotating drum device 11, but also for the emboss-groove treatment device 31 as well. This will be described in detail below.

First, as previously described with reference to FIG. 2, two protruding portions 31p are provided on the outer circumferential surface of the lower roll 31d (corresponding to a second rotating body) of the emboss-groove treatment device 31 at different positions from each other in the rotation direction Dc31 (positions at an angular interval of 180°). Therefore, for each full rotation, the lower roll 31d forms emboss grooves EB for two portions 1p that are to become the napkin 1. For this reason, non-redundant protruding-portion ID numbers are set for the two protruding portions 31p as identifiers for being distinguished from the other protruding portion 31p. Specifically, "1" is set as the protruding-portion ID number for one protruding portion 31p, and "2" is set as the protruding-portion ID number for the other protruding portion 31p. Note that the term "set" used here means the same thing as in the previous description that was given. In other words, in this example, the corresponding protruding-portion ID number is engraved or ink-printed in the vicinity of the protruding portions 31p of the lower roll 31d so as to be visually recognizable.

Also, each time the unit signal of the synchronization signal is output, the lower roll 31d forms one emboss groove EB in one absorbent core 3c with one protruding portion 31p (corresponding to a second treated-portion forming step). Here, each time the unit signal is output, the previously described controller generates and outputs information indicating the protruding-portion ID number of the protruding portion 31p that formed the emboss groove EB in the absorbent core 3c when the unit signal was output. For example, when the unit signal is output, if the protruding portion 31p having the protruding-portion ID number "1" is forming the emboss groove EB in the absorbent core 3c, the controller outputs "1" as the protruding-portion ID number information. And then when the protruding portion 31p having the protruding-portion ID number "2" is forming the emboss groove EB in the absorbent core 3c, the controller outputs "2" as the protruding-portion ID number information.

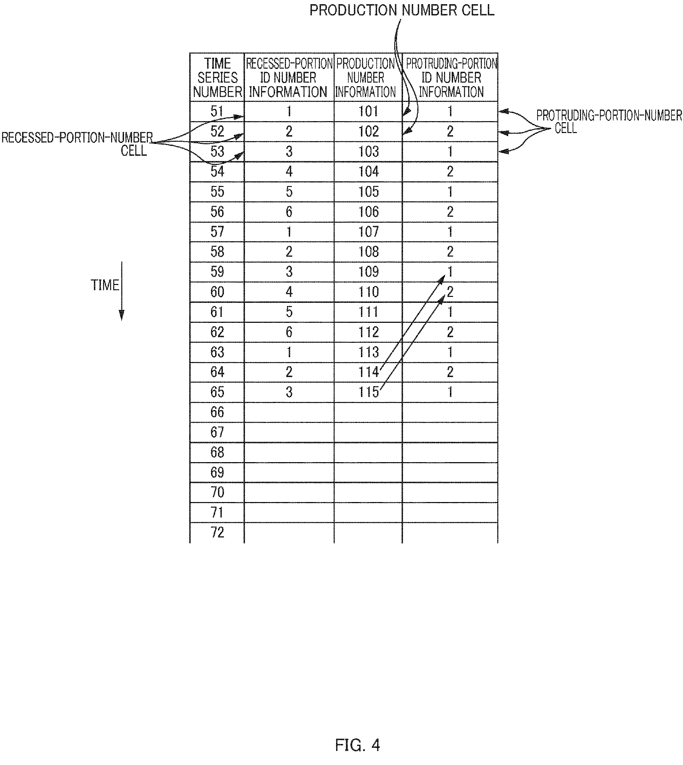

Furthermore, as shown in FIG. 4, the recording area in the memory of the controller further has protruding-portion-number cells in correspondence with the previously described time series numbers, production number cells, and recessed-portion-number cells in FIG. 3. The protruding-portion ID number information is recorded in the protruding-portion-number cells in the order in which the protruding-portion ID number information is output. Specifically, similarly to the previously described recessed-portion ID number information, each time a piece of protruding-portion ID number information is output, that information is recorded in the protruding-portion-number cell that has the smallest time series number among the empty protruding-portion-number cells. Accordingly, a piece of recessed-portion ID number information, a piece of production number information, and a piece of protruding-portion ID number information that correspond to processing performed at the time of the output of the same unit signal are recorded being correlated with the same time series number.

It should be noted that in this case as well, similarly to the description given above, the predetermined direction-of-transport position P31 for forming the emboss groove EB and the predetermined direction-of-transport position P71 for printing the production number are shifted from each other by 5 in total of napkins 1 and portions 1p that are to become napkins 1. For this reason, a piece of production number information and a piece of protruding-portion ID number information that are correlated with the same time series number in the recording area do not indicate the same napkin 1 as each other, that is to say, the correspondence between the production number information and the protruding-portion ID number information recorded in the recording area is shifted from the correct correspondence, more specifically is shifted by 5 in total of napkins 1 and portions 1p, which exist between the two predetermined positions P31 and P71.

In view of this, in this case as well, in order to find the correct correspondence, the controller shifts the correspondence between the production number information and the recessed-portion ID number information in the recording area by 5 places in the time series.

For example, in the case of the information recorded in the recording area shown in FIG. 4, for the production number information "114", the recessed-portion ID number that is 5 places in the past in the time series, namely "1", is acquired as the correct number information, and for the next production number information "115", the recessed-portion ID number that is 5 places in the past in the time series, namely "2", is acquired as the correct number information. Accordingly, it is possible to correctly recognize correlating with each other: the piece of information indicating the production number that was printed on a napkin 1 and the piece of information indicating the protruding-portion ID number of the protruding portion 31p that formed the emboss groove EB in the absorbent core 3c of the napkin 1 that has that production number. As a result, if the defect in the absorbent core 3c was caused by one of the two protruding portions 31p of the lower roll 31d of the emboss-groove treatment device 31, it is possible to specify which of the protruding portions 31p caused the defect.

Note that in the above-described case, there are two devices that can possibly be the cause of the defect, namely the devices 11 and 31. It is possible that both the rotating drum device 11 and the emboss-groove treatment device 31 are causes of defects. For this reason, it is useful to be able to easily specify which of the two devices 11 and 31 is the cause of a defect, but from the above viewpoint, it is desirable that the number of recessed portions 11h, 11h... provided in the rotating drum 11d of the rotating drum device 11 is different from the number of protruding portions 31p provided on the lower roll 31d of the emboss-groove treatment device 31, as in the example in FIG. 2. According to this configuration, if the defective product production pattern is examined by comparing the recessed-portion ID number and protruding-portion ID number information in the recording area shown in FIG. 4 with defective production information (not shown) regarding the napkin 1 provided by the consumer or the like, it is possible to easily examine whether a recessed portion 11h of the rotating drum device 11 or a protruding portion 31p of the emboss-groove treatment device 31 was the cause of the defect.

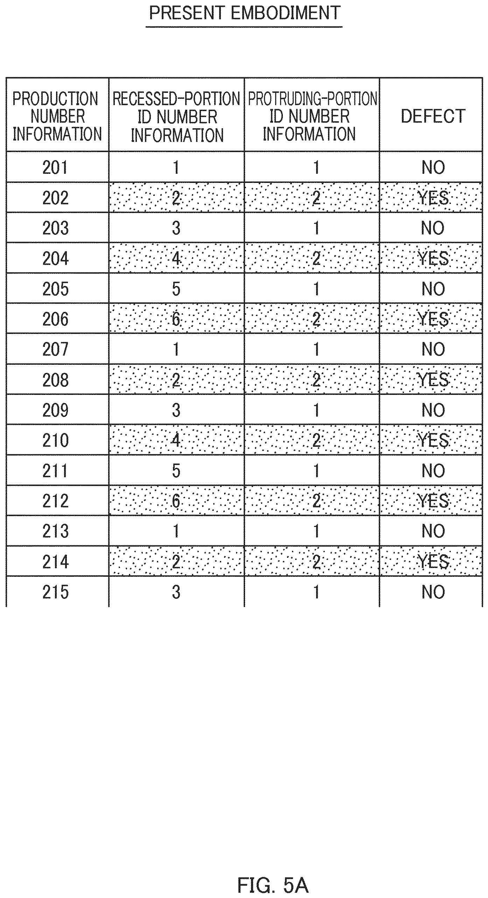

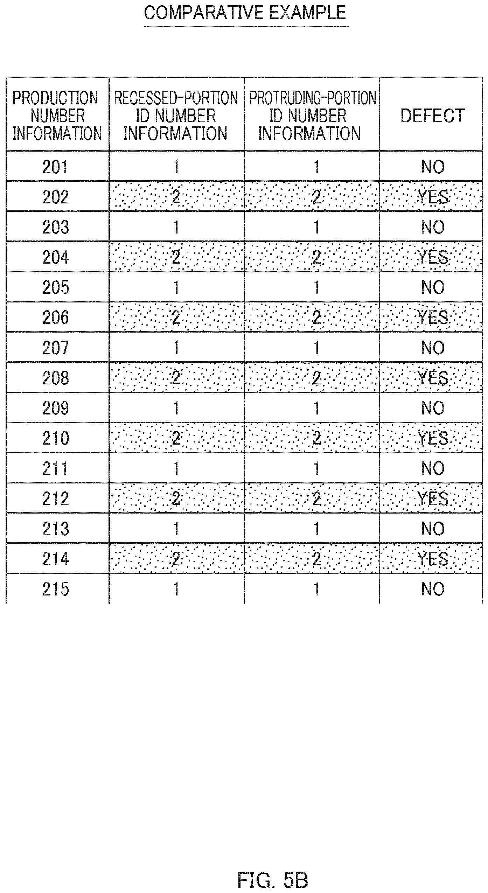

FIGS. 5A and 5B are conceptual diagrams for describing this. Note that both of these diagrams show correct correspondences obtained by correcting the correspondence between the protruding-portion ID numbers and the recessed-portion ID numbers. In this correct correspondence, concerning the recessed portion 11h of the rotating drum device 11 that formed a certain absorbent core 3c and concerning the protruding portion 31p of the emboss-groove treatment device 31 that formed the emboss groove EB in the certain absorbent core 3c, information indicating its recessed-portion ID number is correlated with information indicating its protruding-portion ID number. In other words, these pieces of information function as information by which the recessed portion 11h that formed the certain absorbent core 3c out of the plurality of recessed portions 11h, 11h... is correlated with the protruding portion 31p that formed the emboss groove EB in the certain absorbent core 3c out of the several protruding portions 31p.

FIG. 5A shows an example of the case of the present embodiment, that is to say the case where the number of recessed portions 11h that are provided is different from the number of protruding portions 31p that are provided, whereas FIG. 5B shows a comparative example in which the number of recessed portions 11h that are provided is the same as the number of protruding portions 31p that are provided. The defective product information provided by the consumer for indicating the production number of the napkin 1 that has a defective absorbent core 3c is the same in both of these figures.

As can be seen in FIG. 5B, in the comparative example, the number of provided recessed portions 11h and the number of provided protruding portions 31p are both 2. Also, in this comparative example, in the case where the protruding portion 31p having the protruding-portion ID number "2" has a problem, defective products appear with the production pattern shown in FIG. 5B. In other words, a defective product is produced when the protruding-portion ID number information is "2", and is not produced when the protruding-portion ID number information is "1", whereas a defective product is produced when the recessed-portion ID number information is "2", and is not produced when the recessed-portion ID number information is "1".

Here, when a maintenance/inspection worker for the manufacturing line Lm sees such a production pattern, the worker can determine that at least either the protruding portion 31p having the protruding-portion ID number "2" or the recessed portion 11h having the recessed-portion ID number "2" has a problem. However, the worker cannot determine which of the two has a problem. In other words, it is not possible to specify whether a protruding portion 31p of the emboss-groove treatment device 31 has a problem or a recessed portion 11h of the rotating drum device 11 has a problem.

In contrast, in the case of the present embodiment shown in FIG. 5A (i.e., the case where the number of provided recessed portions 11h is 6 and the number of provided protruding portions 31p is 2, and thus these numbers are different from each other), if the protruding portion 31p having the protruding-portion ID number "2" has a problem, the defective product production pattern shown in FIG. 5A appears. In other words, first, a defective product is produced when the protruding-portion ID number information is "2", and is not produced when the protruding-portion ID number information is "1", whereas a defective product is produced when the recessed-portion ID number information is "2", "4", or "6", and is not produced when the recessed-portion ID number information is "1", "3", or "5".

When a worker see such a production pattern, the worker can determine that either the protruding portion 31p having the protruding-portion ID number "2" has a problem, or that the three recessed portions 11h having the recessed-portion ID numbers "2", "4", and "6" have a problem. At this time, it is unlikely that all three of the recessed portions 11h "2", "4", and "6" have a problem, and therefore the worker can easily infer that the protruding portion 31p "2" probably has a problem. Accordingly, the worker can roughly determine that the protruding portion 31p of the emboss-groove treatment device 31 has a problem.

Note that in order to make it easier to make such an examination of the cause of the problem, it is further desirable that either the number of provided recessed portions 11h or the number of provided protruding portions 31p is a prime number, and the other number is not an integral multiple (excluding 0 and 1) thereof.

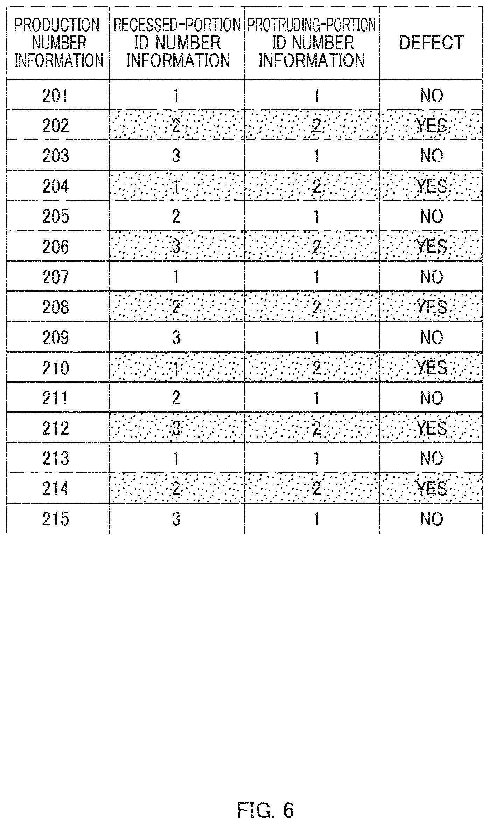

FIG. 6 shows an example of a case that satisfies the above-described relationship between the numbers of provided portions, namely the case where the number of provided recessed portions 11h is "3" and the number of provided protruding portions 31p is "2". In this example, the prime number of provided portions is "2", and the other number of provided portions that is not an integral multiple thereof is "3".

In this case, if the protruding portion 31p having the protruding-portion ID number "2" has a problem, defective products will appear with the production pattern shown in FIG. 6. Specifically, first, a defective product is produced when the protruding-portion ID number information is "2", and is not produced when the protruding-portion ID number information is "1", whereas a defective product is produced when the recessed-portion ID number information is "1", "2", or "3", and there are no cases where a defective product is not produced.

When a worker see such a production pattern, the worker can first determine that either the protruding portion 31p having the protruding-portion ID number "2" has a problem, or that all of the recessed portions 11h, 11h... have a problem. At this time, it is unlikely that all of the recessed portions 11h, 11h... have a problem, and therefore the worker can easily infer that the protruding portion 31p "2" probably has a problem. Accordingly, the worker can roughly determine that the protruding portion 31p of the emboss-groove treatment device 31 has a problem.

### Other embodiments

Although the embodiment of the present disclosure have been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof. For example, modification which will be described below is possible.

In the above embodiment, as shown in FIG. 2, only one cutter blade 15c is provided on the upper roll 15u of the absorbent-body cutter device 15, and similarly, only one protruding portion (not shown) is provided in the lower roll 51d of the round-seal treatment device 51, and only one cutter blade 61c is provided on the upper roll 61u of the die cutter device 61. For this reason, if a problem occurs due to the cutter blades 15c or 61c or the protruding portion, there is no need to specify which of them is the cause of the problem, and therefore in the previously described embodiment, the above-described innovation is not made for the devices 15, 51, and 61. However, there is no limitation whatsoever to this. If a plurality of cutter blades 15c and 61c and protruding portions are provided, the above-described innovation may be made for the devices 15, 51, and 61 as well.

Although the sanitary napkin 1 is illustrated as an example of the absorbent article in the above embodiment, there is no limitation whatsoever to this. For example, the absorbent article may be a pull-on or tape-type disposable diaper, or may be a urine absorbing pad or the like. In other words, any article that absorbs excrement from the wearer is encompassed in the scope of the absorbent article here.

In the above embodiment, as the correlation information for correlating the absorbent articles with the treatment processing sections, used are information indicating production numbers printed on napkins 1 serving as absorbent articles, and information indicating recessed-portion ID numbers set for the recessed portions 11h serving as treatment processing sections. However, there is no limitation whatsoever to this. For example, instead of using information indicating production numbers printed on the napkins 1, it is possible to print production numbers on absorbent cores 3c serving as treated portions, and use information indicating such production numbers as the above-described correlation information. Even with this configuration, it is possible to specify which of the plurality of recessed portions 11h, 11h... of the rotating drum 11d of the rotating drum device 11 is the recessed portion 11h that formed a defective absorbent core 3c.

Although numbers such as production numbers are illustrated as examples of identifiers in the above embodiment, there is no limitation whatsoever to this. Instead of numbers, the identifiers may be text, or symbols other than text. The identifiers may also be graphical, such as a barcode or a QR code. Note that in the case of using a barcode or a QR code, it goes without saying that the code has been assigned the meaning of a non-redundant production number, and that the production number can be recognized by reading the code with a code reader.

An inspection step (not shown) for inspecting the napkin 1, and a discharge step (not shown) in which a napkin 1 determined to be a defective product in the inspection is discharged from the transporting route are not mentioned in the above embodiment, but such steps may be performed in the manufacturing line Lm. Note that in this case, the direction-of-transport positions where these steps are performed are desirably downstream with respect to the direction-of-transport position where the production-number printing step S70 is performed. According to this configuration, a production number is printed on a defective napkin 1 as well. Accordingly, it is possible to more quickly examine the cause of the problem based on the production number.

For example, before a defective napkin 1 reaches the consumer, it is possible to swiftly specify, based on the production number of the defective product discharged in the discharging step, the recessed portion 11h of the rotating drum device 11 that formed the absorbent core 3c of the defective product. Accordingly, it is possible to quickly inspect and repair that recessed portion 11h, and as a result, it is possible to effectively prevent the further production of defective products.

In the above embodiment, no mention is made of whether or not the manufacturing line Lm has backup rotating drum devices 11. But, in some cases, the manufacturing line Lm may have at least one backup device that has similar specifications to the rotating drum device 11 that is currently running. According to this configuration, when a problem occurs with the rotating drum device 11 that is currently running in the manufacturing line Lm, it can be swiftly replaced with the backup rotating drum device 11, thus suppressing downtime in the manufacturing line Lm. It should be noted that in this case, in order for the above-described innovation to be able to be made even when the device is replaced with the backup rotating drum device 11, it goes without saying that recessed-portion ID numbers are set for the recessed portions 11h, 11h... of the rotating drum 11d of the backup rotating drum device 11 similarly to the description given above.

Furthermore, in some cases, the specifications of the recessed portion 11h of the backup device (e.g., the size and number of provided recessed portions 11h) may be different from the rotating drum device 11 that is currently running. In such as a case, the backup rotating drum device 11 can be used to manufacture an absorbent core 3c that has different specifications than the absorbent core 3c being formed by the rotating drum device 11 that is currently running.

Although no mention is made of a step in which a plurality of napkins 1, 1... are ultimately placed in a packaging bag body (not shown) and shipped in the above embodiment, such a step may be performed. Note that in this case, processing for placing a plurality of napkins 1, 1... in a packaging bag body is performed at a position downstream with respect to the direction-of-transport position at which the production-number printing step S70 is performed. In this case, the following configuration is desirable.

First, an appropriate printing device such as an inkjet printer prints non-redundant production numbers (hereinafter, also called bag-body production numbers) on the packaging bag bodies in order to be distinguished from other packaging bag bodies. Specifically, the controller generates bag-body production number information (corresponding to second information) for each packaging bag body (corresponding to a second information generating step) and outputs the information to the printer, and then the bag-body production numbers are printed on the packaging bag bodies based on such information. In parallel with this, the aforementioned controller records information indicating the printed bag-body production numbers in bag-body production number cells separately provided in the recording area of the memory in association with the production numbers of the napkins 1 that were placed in the packaging bag body.

According to this configuration, if a defective napkin 1 is discovered at a later time, it is possible to swiftly specify the bag-body production number of the packaging bag body that includes that napkin 1, based on the production number of the defective product. Accordingly, it is possible to collect defective products together with one packaging bag body at a time rather than collecting defective napkins 1 individually, thus making it possible to increase efficiency in the collection of such napkins 1.

In the above embodiment, the unit signals of the synchronization signal are generated by the processor reading out a program stored in the memory of the controller and executing the program, but there is no limitation whatsoever to this. For example, the controller may have an electric circuit that generates the synchronization signal, or the synchronization signal may be generated by using a servomotor to rotate a rotary encoder.

### [Reference Signs List]

1 sanitary napkin (absorbent article), 1a semi-finished goods,
1e outer edge portion, 1p portion to become a napkin (portion to become an absorbent article),
1s seal portion,
2 top sheet, 2a top sheet, (top sheet continuous sheet),
3 absorbent body, 3a absorbent body continuous body, 3ae end portion,
3c absorbent core (treated portion, first treated portion),
3t tissue paper,
3ta tissue paper (tissue paper continuous sheet, continuous sheet),
3tC material coil,
4 back sheet, 4a back sheet (back sheet continuous sheet),
11 rotating drum device (treated-portion forming device),
11d rotating drum (rotating body, first rotating body),
11h recessed portion (treatment processing section, first treatment processing section),
12 duct,
13 folding guide member,
15 absorbent-body cutter device, 15u upper roll, 15d lower roll, 15c cutter blade,
21 supply roll,
31 emboss-groove treatment device,
31u upper roll,
31d lower roll (second rotating body), 31p protruding portion (second treatment processing section),
41 supply roll,
51 round-seal treatment device,
51u upper roll, 51d lower roll,
61 die cutter device, 61u upper roll, 61d lower roll,
61c cutter blade,
71 inkjet printer (printing device),
CV transporting mechanism (transporting device),
EB emboss groove (second treated portion),
Lm manufacturing line,
RL feeding device,
P11 predetermined position (first predetermined position), P31 predetermined position (second predetermined position),
P71 predetermined position (third predetermined position),
S10 absorbent-body forming step , S20 top-sheet supplying step,
S30 emboss-groove treatment step, S40 back-sheet supplying step,
S50 round-seal treatment step, S60 dividing step, S70 production-number printing step

## Claims

1. A method for manufacturing an absorbent article, comprising:
a transporting step of transporting a continuous sheet in a direction of transport,
the continuous sheet being continuous in the direction of transport;
a treated-portion forming step of repeatedly forming a treated portion in a portion of the continuous sheet in the direction of transport,
the portion being a unit portion that is to become the absorbent article,
the treated-portion forming step being performed at a predetermined position in the direction of transport,
the treated-portion forming step including forming the treated portions by a plurality of treatment processing sections,
the plurality of treatment processing sections being provided at different positions in a rotation direction on an outer circumferential surface of a rotating body,
the rotating body being arranged at the predetermined position and rotating along the direction of transport; and
an information generating step of generating information regarding either of each treated portion and the absorbent article that has that treated portion,
the information generating step including generating, as the information, correlation information for correlating either of each treated portion and each absorbent article having that treated portion with the treatment processing section which has formed that treated portion among the plurality of treatment processing sections,
wherein
rotation of the rotating body and transporting of the continuous sheet are coordinated with each other based on a synchronization signal constituted by a unit signal that is repeatedly output,
each time the unit signal is output, only one of the portions that are to become the absorbent articles in the continuous sheet is transported in the direction of transport,
a treatment-processing-section identifier is non-redundantly set for each treatment processing section,
each time the unit signal is output,
the rotating body rotates such that a treated portion is formed by one of the treatment processing sections, and
information indicating the treatment-processing-section identifier of the one treatment processing section which has formed that treated portion is output as the correlation information,
at a third predetermined position in the direction of transport, each time the unit signal is output,
the absorbent article or a portion to become the absorbent article passes the third predetermined position,
an identifier is non-redundantly provided on the passing absorbent article or portion to become the absorbent article, and
information indicating the identifier is output as the correlation information, and
the information indicating the identifier and the information indicating the treatment-processing-section identifier are correlated with each other based on a sum of a number of absorbent articles and a number of portions to become the absorbent article,
the absorbent articles and the portions being those existing between the predetermined position and the third predetermined position.

2. A method for manufacturing an absorbent article, comprising:
a transporting step of transporting a continuous sheet in a direction of transport,
the continuous sheet being continuous in the direction of transport;
a treated-portion forming step of repeatedly forming a treated portion in a portion of the continuous sheet in the direction of transport,
the portion being a unit portion that is to become the absorbent article,
the treated-portion forming step being performed at a predetermined position in the direction of transport,
the treated-portion forming step including forming the treated portions by a plurality of treatment processing sections,
the plurality of treatment processing sections being provided at different positions in a rotation direction on an outer circumferential surface of a rotating body,
the rotating body being arranged at the predetermined position and rotating along the direction of transport; and
an information generating step of generating information regarding either of each treated portion and the absorbent article that has that treated portion,
the information generating step including generating, as the information, correlation information for correlating either of each treated portion and each absorbent article having that treated portion with the treatment processing section which has formed that treated portion among the plurality of treatment processing sections,
wherein
letting the treated portions be first treated portions, the rotating body be a first rotating body, the predetermined position be a first predetermined position, and the treatment processing sections be first treatment processing sections,
the first treatment processing sections are provided at a first angular interval in the rotation direction,
the method further comprises a second treated-portion forming step of forming a second treated portion in each first treated portion,
the second treated-portion forming step is performed at a second predetermined position in the direction of transport that is different from the first predetermined position,
the second treated-portion forming step includes forming the second treated portions by several second treatment processing sections,
the several second treatment processing sections are provided at a second angular interval in a rotation direction on an outer circumferential surface of a second rotating body,
the second rotating body being arranged at the second predetermined position and rotating along the direction of transport,
the correlation information is information for correlating a first treatment processing section with a second treatment processing section,
the first treatment processing section being one of the plurality of first treatment processing sections and forming a certain first treated portion,
the second treatment processing section being one of the several second treatment processing sections and forming the second treated portion in the certain first treated portion,
the number of first treatment processing sections provided on the first rotating body is different from the number of second treatment processing sections provided on the second rotating body,
either of the number of first treatment processing sections provided on the first rotating body and the number of second treatment processing sections provided on the second rotating body is a prime number, and
the other one of the numbers is not an integral multiple (excluding 0 and 1) of the prime number.

3. The method for manufacturing an absorbent article according to claim 1, wherein
among the absorbent articles, an absorbent article determined to be a defective product is discharged at a position downstream in the direction of transport with respect to the third predetermined position.

4. The method for manufacturing an absorbent article according to any of claims 1 to 3, wherein
the method further comprises:
a step of placing a plurality of the absorbent articles into one packaging bag body; and
a second information generating step of generating second information,
the second information being information for correlating the plurality of absorbent articles with the packaging bag body into which the absorbent articles were placed.

5. A device for manufacturing an absorbent article, comprising:
a transporting device configured to transport a continuous sheet in a direction of transport,
the continuous sheet being continuous in the direction of transport;
a treated-portion forming device configured to repeatedly form a treated portion in a portion of the continuous sheet in the direction of transport,
the portion being a unit portion that is to become the absorbent article,
the forming being performed at a predetermined position in the direction of transport,
the treated-portion forming device forming the treated portions by a plurality of treatment processing sections,
the plurality of treatment processing sections being provided at different positions in a rotation direction on an outer circumferential surface of a rotating body,
the rotating body being arranged at the predetermined position in the direction of transport and rotating along the direction of transport; and
an information generation device configured to generate information regarding either of each treated portion and the absorbent article that has that treated portion,
the information generation device generating, as the information, correlation information for correlating either of each treated portion and each absorbent article having that treated portion with the treatment processing section which has formed that treated portion among the plurality of treatment processing sections,
wherein
rotation of the rotating body and transporting of the continuous sheet are coordinated with each other based on a synchronization signal constituted by a unit signal that is repeatedly output,
each time the unit signal is output, only one of the portions that are to become the absorbent articles in the continuous sheet is transported in the direction of transport,
a treatment-processing-section identifier is non-redundantly set for each treatment processing section,
each time the unit signal is output,
the rotating body rotates such that a treated portion is formed by one of the treatment processing sections, and
information indicating the treatment-processing-section identifier of the one treatment processing section which has formed that treated portion is output as the correlation information,
at a third predetermined position in the direction of transport, each time the unit signal is output,
the absorbent article or a portion to become the absorbent article passes the third predetermined position,
an identifier is non-redundantly provided on the passing absorbent article or portion to become the absorbent article, and
information indicating the identifier is output as the correlation information, and
the information indicating the identifier and the information indicating the treatment-processing-section identifier are correlated with each other based on a sum of a number of absorbent articles and a number of portions to become the absorbent article,
the absorbent articles and the portions being those existing between the predetermined position and the third predetermined position.

6. A device for manufacturing an absorbent article, comprising:
a transporting device configured to transport a continuous sheet in a direction of transport,
the continuous sheet being continuous in the direction of transport;
a treated-portion forming device configured to repeatedly form a treated portion in a portion of the continuous sheet in the direction of transport,
the portion being a unit portion that is to become the absorbent article,
the forming being performed at a predetermined position in the direction of transport,
the treated-portion forming device forming the treated portions by a plurality of treatment processing sections,
the plurality of treatment processing sections being provided at different positions in a rotation direction on an outer circumferential surface of a rotating body,
the rotating body being arranged at the predetermined position in the direction of transport and rotating along the direction of transport; and
an information generation device configured to generate information regarding either of each treated portion and the absorbent article that has that treated portion,
the information generation device generating, as the information, correlation information for correlating either of each treated portion and each absorbent article having that treated portion with the treatment processing section which has formed that treated portion among the plurality of treatment processing sections,
wherein
letting the treated portions be first treated portions, the rotating body be a first rotating body, the predetermined position be a first predetermined position, and the treatment processing sections be first treatment processing sections,
the first treatment processing sections are provided at a first angular interval in the rotation direction,
the method further comprises a second treated-portion forming step of forming a second treated portion in each first treated portion,
the second treated-portion forming step is performed at a second predetermined position in the direction of transport that is different from the first predetermined position,
the second treated-portion forming step includes forming the second treated portions by several second treatment processing sections,
the several second treatment processing sections are provided at a second angular interval in a rotation direction on an outer circumferential surface of a second rotating body,
the second rotating body being arranged at the second predetermined position and rotating along the direction of transport,
the correlation information is information for correlating a first treatment processing section with a second treatment processing section,
the first treatment processing section being one of the plurality of first treatment processing sections and forming a certain first treated portion,
the second treatment processing section being one of the several second treatment processing sections and forming the second treated portion in the certain first treated portion,
the number of first treatment processing sections provided on the first rotating body is different from the number of second treatment processing sections provided on the second rotating body,
either of the number of first treatment processing sections provided on the first rotating body and the number of second treatment processing sections provided on the second rotating body is a prime number, and
the other one of the numbers is not an integral multiple (excluding 0 and 1) of the prime number.

7. The device for manufacturing an absorbent article according to claim 5, wherein
among the absorbent articles, an absorbent article determined to be a defective product is discharged at a position downstream in the direction of transport with respect to the third predetermined position.

## Patentansprüche

1. Verfahren für die Herstellung eines absorbierenden Artikels, das Folgendes umfasst:
einen Transportschritt des Transportierens einer fortlaufenden Lage in einer Transportrichtung,
wobei die fortlaufende Lage in der Transportrichtung fortlaufend ist;
einen Bildungsschritt eines behandelten Teils des wiederholten Bildens eines behandelten Teils in einem Teil der fortlaufenden Lage in der Transportrichtung,
wobei das Teil ein Einheitsteil ist, das der absorbierende Artikel wird,
der Bildungsschritt eines behandelten Teils an einer vorgegebenen Position in der Transportrichtung durchgeführt wird,
der Bildungsschritt eines behandelten Teils das Bilden der behandelten Teile durch eine Vielzahl von Behandlungsbearbeitungsabschnitten einschließt,
wobei die Vielzahl von Behandlungsbearbeitungsabschnitten an verschiedenen Positionen in einer Rotationsrichtung auf einer äußeren Umfangsoberfläche eines rotierenden Körpers bereitgestellt ist,
der rotierende Körper an der vorgegebenen Position angeordnet ist und entlang der Transportrichtung rotiert; und
einen Informationserzeugungsschritt des Erzeugens von Information bezüglich eines von jedem behandelten Teil und dem absorbierenden Artikel, der das behandelte Teil aufweist,
wobei der Informationserzeugungsschritt das Erzeugen, als Information, von Korrelationsinformation für das Korrelieren von einem von jedem behandelten Teil und jedem absorbierenden Artikel, der das behandelte Teil aufweist, mit dem Behandlungsbearbeitungsabschnitt, der das behandelte Teil gebildet hat, unter der Vielzahl von Behandlungsbearbeitungsabschnitten einschließt,
wobei
Rotation des rotierenden Körpers und Transport der fortlaufenden Lage miteinander basierend auf einem Synchronisationssignal, das aus einem Einheitssignal besteht, das wiederholt ausgegeben wird, koordiniert werden,
jedes Mal, wenn das Einheitssignal ausgegeben wird, nur eines der Teile, die die absorbierenden Artikel werden, in der fortlaufenden Lage in der Transportrichtung transportiert wird,
ein Identifikator des Behandlungsbearbeitungsabschnitts nichtredundant für jeden Behandlungsbearbeitungsabschnitt eingestellt ist,
jedes Mal, wenn das Einheitssignal ausgegeben wird,
der rotierende Körper derart rotiert, dass ein behandeltes Teil durch einen der Behandlungsbearbeitungsabschnitte gebildet wird, und
Information, die den Identifikator des Behandlungsbearbeitungsabschnitts des einen Behandlungsbearbeitungsabschnitts anzeigt, der das behandelte Teil gebildet hat, als die Korrelationsinformation ausgegeben wird,
an einer dritten vorgegebenen Position in der Transportrichtung jedes Mal, wenn das Einheitssignal ausgegeben wird,
der absorbierende Artikel oder ein Teil, das der absorbierende Artikel wird, die dritte vorgegebene Position passiert,
ein Identifikator nichtredundant auf dem passierenden absorbierenden Artikel oder Teil, das der absorbierende Artikel wird, bereitgestellt wird und
Information, die den Identifikator anzeigt, als die Korrelationsinformation ausgegeben wird, und
die Information, die den Identifikator anzeigt, und die Information, die den Identifikator des Behandlungsbearbeitungsabschnitts anzeigt, miteinander basierend auf einer Summe einer Anzahl absorbierender Artikel und einer Anzahl von Teilen, die der absorbierende Artikel werden, korreliert werden,
wobei die absorbierenden Artikel und die Teile, die diese sind, zwischen der vorgegebenen Position und der dritten vorgegebenen Position vorliegen.

2. Verfahren für die Herstellung eines absorbierenden Artikels, das Folgendes umfasst:
einen Transportschritt des Transportierens einer fortlaufenden Lage in einer Transportrichtung,
wobei die fortlaufende Lage in der Transportrichtung fortlaufend ist;
einen Bildungsschritt eines behandelten Teils des wiederholten Bildens eines behandelten Teils in einem Teil der fortlaufenden Lage in der Transportrichtung,
wobei das Teil ein Einheitsteil ist, das der absorbierende Artikel wird,
der Bildungsschritt eines behandelten Teils an einer vorgegebenen Position in der Transportrichtung durchgeführt wird,
der Bildungsschritt eines behandelten Teils das Bilden der behandelten Teile durch eine Vielzahl von Behandlungsbearbeitungsabschnitten einschließt,
wobei die Vielzahl von Behandlungsbearbeitungsabschnitten an verschiedenen Positionen in einer Rotationsrichtung auf einer äußeren Umfangsoberfläche eines rotierenden Körpers bereitgestellt ist,
der rotierende Körper an der vorgegebenen Position angeordnet ist und entlang der Transportrichtung rotiert; und
einen Informationserzeugungsschritt des Erzeugens von Information bezüglich eines von jedem behandelten Teil und dem absorbierenden Artikel, der das behandelte Teil aufweist,
wobei der Informationserzeugungsschritt das Erzeugen, als Information, von Korrelationsinformation für das Korrelieren von einem von jedem behandelten Teil und jedem absorbierenden Artikel, der das behandelte Teil aufweist, mit dem Behandlungsbearbeitungsabschnitt, der das behandelte Teil gebildet hat, unter der Vielzahl von Behandlungsbearbeitungsabschnitten einschließt,
wobei,
wenn die behandelten Teile erste behandelte Teile sind, der rotierende Körper ein erster rotierender Körper ist, die vorgegebene Position eine erste vorgegebene Position ist und die Behandlungsbearbeitungsabschnitte erste Behandlungsbearbeitungsabschnitte sind,
die ersten Behandlungsbearbeitungsabschnitte an einem ersten Winkelintervall in der Rotationsrichtung bereitgestellt sind,
das Verfahren weiter einen zweiten Bildungsschritt eines behandelten Teils des Bildens eines zweiten behandelten Teils in jedem ersten behandelten Teil umfasst,
der zweite Bildungsschritt eines behandelten Teils an einer zweiten vorgegebenen Position in der Transportrichtung durchgeführt wird, die sich von der ersten vorgegebenen Position unterscheidet,
der zweite Bildungsschritt eines behandelten Teils das Bilden der zweiten behandelten Teile durch mehrere zweite Behandlungsbearbeitungsabschnitte einschließt,
die mehreren zweiten Behandlungsbearbeitungsabschnitte an einem zweiten Winkelintervall in einer Rotationsrichtung auf einer äußeren Umfangsoberfläche eines zweiten rotierenden Körpers bereitgestellt sind,
der zweite rotierende Körper an der zweiten vorgegebenen Position angeordnet ist und entlang der Transportrichtung rotiert,
die Korrelationsinformation Information für das Korrelieren eines ersten Behandlungsbearbeitungsabschnitts mit einem zweiten Behandlungsbearbeitungsabschnitt ist,
wobei der erste Behandlungsbearbeitungsabschnitt einer der Vielzahl von ersten Behandlungsbearbeitungsabschnitten ist und ein bestimmtes erstes behandeltes Teil bildet,
der zweite Behandlungsbearbeitungsabschnitt einer der mehreren zweiten Behandlungsbearbeitungsabschnitte ist und das zweite behandelte Teil in dem bestimmten ersten behandelten Teil bildet,
sich die Anzahl der ersten Behandlungsbearbeitungsabschnitte, die auf dem ersten rotierenden Körper bereitgestellt sind, von der Anzahl der zweiten Behandlungsbearbeitungsabschritte, die auf dem zweiten rotierenden Körper bereitgestellt sind, unterscheidet,
eine der Anzahl der ersten Behandlungsbearbeitungsabschnitte, die auf dem ersten rotierenden Körper bereitgestellt sind, und der Anzahl der zweiten Behandlungsbearbeitungsabschritte, die auf dem zweiten rotierenden Körper bereitgestellt sind, eine Primzahl ist und
die andere der Anzahlen kein ganzzahliges Mehrfaches (ausschließlich 0 und 1) der Primzahl ist.

3. Verfahren für die Herstellung eines absorbierenden Artikels nach Anspruch 1, wobei
unter den absorbierenden Artikeln ein absorbierender Artikel, der als ein fehlerhaftes Produkt bestimmt wurde, an einer Position, in der Transportrichtung in Bezug auf die dritte vorgegebene Position nachgelagert, ausgesondert wird.

4. Verfahren für die Herstellung eines absorbierenden Artikels nach einem der Ansprüche 1 bis 3, wobei
das Verfahren weiter Folgendes umfasst:
einen Schritt des Einbringens einer Vielzahl der absorbierenden Artikel in einen Verpackungsbeutelkörper; und
einen zweiten Informationserzeugungsschritt des Erzeugens von zweiter Information,
wobei die zweite Information Information für das Korrelieren der Vielzahl von absorbierenden Artikeln mit dem Verpackungsbeutelkörper ist, in den die absorbierenden Artikel eingebracht wurden.

5. Vorrichtung für die Herstellung eines absorbierenden Artikels, die Folgendes umfasst:
eine Transportvorrichtung, die zum Transport einer fortlaufenden Lage in einer Transportrichtung konfiguriert ist,
wobei die fortlaufende Lage in der Transportrichtung fortlaufend ist;
eine Bildungsvorrichtung eines behandelten Teils, die zum wiederholten Bilden eines behandelten Teils in einem Teil der fortlaufenden Lage in der Transportrichtung konfiguriert ist,
wobei das Teil ein Einheitsteil ist, das der absorbierende Artikel wird,
das Bilden an einer vorgegebenen Position in der Transportrichtung durchgeführt wird,
die Bildungsvorrichtung eines behandelten Teils das Bilden der behandelten Teile durch eine Vielzahl von Behandlungsbearbeitungsabschnitten bildet,
wobei die Vielzahl von Behandlungsbearbeitungsabschnitten an verschiedenen Positionen in einer Rotationsrichtung auf einer äußeren Umfangsoberfläche eines rotierenden Körpers bereitgestellt ist,
der rotierende Körper an der vorgegebenen Position in der Transportrichtung angeordnet ist und entlang der Transportrichtung rotiert; und
eine Informationserzeugungsvorrichtung, die zum Erzeugen von Information bezüglich eines von jedem behandelten Teil und dem absorbierenden Artikel, der das behandelte Teil aufweist, konfiguriert ist,
wobei die Informationserzeugungsvorrichtung als Information Korrelationsinformation für das Korrelieren von einem von jedem behandelten Teil und jedem absorbierenden Artikel, der das behandelte Teil aufweist, mit dem Behandlungsbearbeitungsabschnitt, der das behandelte Teil gebildet hat, unter der Vielzahl von Behandlungsbearbeitungsabschnitten erzeugt,
wobei
Rotation des rotierenden Körpers und Transport der fortlaufenden Lage miteinander basierend auf einem Synchronisationssignal, das aus einem Einheitssignal besteht, das wiederholt ausgegeben wird, koordiniert werden,
jedes Mal, wenn das Einheitssignal ausgegeben wird, nur eines der Teile, die die absorbierenden Artikel werden, in der fortlaufenden Lage in der Transportrichtung transportiert wird,
ein Identifikator des Behandlungsbearbeitungsabschnitts nichtredundant für jeden Behandlungsbearbeitungsabschnitt eingestellt ist,
jedes Mal, wenn das Einheitssignal ausgegeben wird,
der rotierende Körper derart rotiert, dass ein behandeltes Teil durch einen der Behandlungsbearbeitungsabschnitte gebildet wird, und
Information, die den Identifikator des Behandlungsbearbeitungsabschnitts des einen Behandlungsbearbeitungsabschnitts anzeigt, der das behandelte Teil gebildet hat, als die Korrelationsinformation ausgegeben wird,
an einer dritten vorgegebenen Position in der Transportrichtung jedes Mal, wenn das Einheitssignal ausgegeben wird,
der absorbierende Artikel oder ein Teil, das der absorbierende Artikel wird, die dritte vorgegebene Position passiert,
ein Identifikator nichtredundant auf dem passierenden absorbierenden Artikel oder Teil, das der absorbierende Artikel wird, bereitgestellt wird und
Information, die den Identifikator anzeigt, als die Korrelationsinformation ausgegeben wird, und
die Information, die den Identifikator anzeigt, und die Information, die den Identifikator des Behandlungsbearbeitungsabschnitts anzeigt, miteinander basierend auf einer Summe einer Anzahl absorbierender Artikel und einer Anzahl von Teilen, die der absorbierende Artikel werden, korreliert werden,
wobei die absorbierenden Artikel und die Teile, die diese sind, zwischen der vorgegebenen Position und der dritten vorgegebenen Position vorliegen.

6. Vorrichtung für die Herstellung eines absorbierenden Artikels, die Folgendes umfasst:
eine Transportvorrichtung, die zum Transport einer fortlaufenden Lage in einer Transportrichtung konfiguriert ist,
wobei die fortlaufende Lage in der Transportrichtung fortlaufend ist;
eine Bildungsvorrichtung eines behandelten Teils, die zum wiederholten Bilden eines behandelten Teils in einem Teil der fortlaufenden Lage in der Transportrichtung konfiguriert ist,
wobei das Teil ein Einheitsteil ist, das der absorbierende Artikel wird,
das Bilden an einer vorgegebenen Position in der Transportrichtung durchgeführt wird,
die Bildungsvorrichtung eines behandelten Teils das Bilden der behandelten Teile durch eine Vielzahl von Behandlungsbearbeitungsabschnitten bildet,
wobei die Vielzahl von Behandlungsbearbeitungsabschnitten an verschiedenen Positionen in einer Rotationsrichtung auf einer äußeren Umfangsoberfläche eines rotierenden Körpers bereitgestellt ist,
der rotierende Körper an der vorgegebenen Position in der Transportrichtung angeordnet ist und entlang der Transportrichtung rotiert; und
eine Informationserzeugungsvorrichtung, die zum Erzeugen von Information bezüglich eines von jedem behandelten Teil und dem absorbierenden Artikel, der das behandelte Teil aufweist, konfiguriert ist,
wobei die Informationserzeugungsvorrichtung als Information Korrelationsinformation für das Korrelieren von einem von jedem behandelten Teil und jedem absorbierenden Artikel, der das behandelte Teil aufweist, mit dem Behandlungsbearbeitungsabschnitt, der das behandelte Teil gebildet hat, unter der Vielzahl von Behandlungsbearbeitungsabschnitten erzeugt,
wobei,
wenn die behandelten Teile erste behandelte Teile sind, der rotierende Körper ein erster rotierender Körper ist, die vorgegebene Position eine erste vorgegebene Position ist und die Behandlungsbearbeitungsabschnitte erste Behandlungsbearbeitungsabschnitte sind,
die ersten Behandlungsbearbeitungsabschnitte an einem ersten Winkelintervall in der Rotationsrichtung bereitgestellt sind,
das Verfahren weiter einen zweiten Bildungsschritt eines behandelten Teils des Bildens eines zweiten behandelten Teils in jedem ersten behandelten Teil umfasst,
der zweite Bildungsschritt eines behandelten Teils an einer zweiten vorgegebenen Position in der Transportrichtung durchgeführt wird, die sich von der ersten vorgegebenen Position unterscheidet,
der zweite Bildungsschritt eines behandelten Teils das Bilden der zweiten behandelten Teile durch mehrere zweite Behandlungsbearbeitungsabschnitte einschließt,
die mehreren zweiten Behandlungsbearbeitungsabschnitte an einem zweiten Winkelintervall in einer Rotationsrichtung auf einer äußeren Umfangsoberfläche eines zweiten rotierenden Körpers bereitgestellt sind,
der zweite rotierende Körper an der zweiten vorgegebenen Position angeordnet ist und entlang der Transportrichtung rotiert,
die Korrelationsinformation Information für das Korrelieren eines ersten Behandlungsbearbeitungsabschnitts mit einem zweiten Behandlungsbearbeitungsabschnitt ist,
wobei der erste Behandlungsbearbeitungsabschnitt einer der Vielzahl von ersten Behandlungsbearbeitungsabschnitten ist und ein bestimmtes erstes behandeltes Teil bildet,
der zweite Behandlungsbearbeitungsabschnitt einer der mehreren zweiten Behandlungsbearbeitungsabschnitte ist und das zweite behandelte Teil in dem bestimmten ersten behandelten Teil bildet,
sich die Anzahl der ersten Behandlungsbearbeitungsabschnitte, die auf dem ersten rotierenden Körper bereitgestellt sind, von der Anzahl der zweiten Behandlungsbearbeitungsabschritte, die auf dem zweiten rotierenden Körper bereitgestellt sind, unterscheidet,
eine der Anzahl der ersten Behandlungsbearbeitungsabschnitte, die auf dem ersten rotierenden Körper bereitgestellt sind, und der Anzahl der zweiten Behandlungsbearbeitungsabschritte, die auf dem zweiten rotierenden Körper bereitgestellt sind, eine Primzahl ist und
die andere der Anzahlen kein ganzzahliges Mehrfaches (ausschließlich 0 und 1) der Primzahl ist.

7. Vorrichtung für die Herstellung eines absorbierenden Artikels nach Anspruch 5, wobei
unter den absorbierenden Artikeln ein absorbierender Artikel, der als ein fehlerhaftes Produkt bestimmt wurde, an einer Position, in der Transportrichtung in Bezug auf die dritte vorgegebene Position nachgelagert, ausgesondert wird.

## Revendications

1. Procédé de fabrication d'un article absorbant, comportant :
une étape de transport consistant à transporter une feuille continue dans une direction de transport,
la feuille continue étant continue dans la direction de transport ;
une étape de formation de partie traitée consistant à former de manière répétée une partie traitée dans une partie de la feuille continue dans la direction de transport,
la partie étant une partie d'unité qui est destinée à devenir l'article absorbant,
l'étape de formation de partie traitée étant effectuée au niveau d'une position prédéterminée dans la direction de transport,
l'étape de formation de partie traitée comprenant l'étape consistant à former les parties traitées par une pluralité de sections de réalisation de traitement,
les sections de la pluralité de sections de réalisation de traitement étant mises en œuvre au niveau de différentes positions dans une direction de rotation sur une surface circonférentielle extérieure d'un corps rotatif,
le corps rotatif étant agencé au niveau de la position prédéterminée et tournant le long de la direction de transport ; et
une étape de génération d'informations consistant à générer des informations se rapportant à l'un ou l'autre parmi chaque partie traitée et l'article absorbant qui a cette partie traitée,
l'étape de génération d'informations comprenant l'étape consistant à générer, pour tenir lieu d'informations, des informations de corrélation servant à corréler l'un ou l'autre parmi chaque partie traitée et chaque article absorbant ayant cette partie traitée avec la section de réalisation de traitement qui a formé cette partie traitée parmi la pluralité de sections de réalisation de traitement,
dans lequel
la rotation du corps rotatif et le transport de la feuille continue sont coordonnés l'une par rapport à l'autre en fonction d'un signal de synchronisation constitué par un signal d'unité qui est émis en sortie de manière répétée,
à chaque fois que le signal d'unité est émis en sortie, uniquement l'une des parties qui sont destinées à devenir les articles absorbants dans la feuille continue est transportée dans la direction de transport,
un identifiant de section de réalisation de traitement est réglé de manière non redondante pour chaque section de réalisation de traitement,
à chaque fois que le signal d'unité est émis en sortie,
le corps rotatif tourne de telle sorte qu'une partie traitée est formée par l'une des sections de réalisation de traitement, et
des informations indiquant l'identifiant de section de réalisation de traitement de ladite une section de réalisation de traitement qui a formé cette partie traitée sont émises en sortie pour tenir lieu d'informations de corrélation,
au niveau d'une troisième position prédéterminée dans la direction de transport, à chaque fois que le signal d'unité est émis en sortie,
l'article absorbant ou une partie destinée à devenir l'article absorbant passe par la troisième position prédéterminée,
un identifiant est fourni de manière non redondante sur l'article absorbant ou la partie destinée à devenir l'article absorbant qui passe, et
des informations indiquant l'identifiant sont émises en sortie pour tenir lieu d'informations de corrélation, et
les informations indiquant l'identifiant et les informations indiquant l'identifiant de section de réalisation de traitement sont corrélées les unes avec les autres en fonction d'une somme d'un nombre d'articles absorbants et d'un nombre de parties destinées à devenir l'article absorbant,
les articles absorbants et les parties étant ceux qui existent entre la position prédéterminée et la troisième position prédéterminée.

2. Procédé de fabrication d'un article absorbant, comportant :
une étape de transport consistant à transporter une feuille continue dans une direction de transport,
la feuille continue étant continue dans la direction de transport ;
une étape de formation de partie traitée consistant à former de manière répétée une partie traitée dans une partie de la feuille continue dans la direction de transport,
la partie étant une partie d'unité qui est destinée à devenir l'article absorbant,
l'étape de formation de partie traitée étant effectuée au niveau d'une position prédéterminée dans la direction de transport,
l'étape de formation de partie traitée comprenant l'étape consistant à former les parties traitées par une pluralité de sections de réalisation de traitement,
les sections de la pluralité de sections de réalisation de traitement étant mises en œuvre au niveau de différentes positions dans une direction de rotation sur une surface circonférentielle extérieure d'un corps rotatif,
le corps rotatif étant agencé au niveau de la position prédéterminée et tournant le long de la direction de transport ; et
une étape de génération d'informations consistant à générer des informations se rapportant à l'un ou l'autre parmi chaque partie traitée et l'article absorbant qui a cette partie traitée,
l'étape de génération d'informations comprenant l'étape consistant à générer, pour tenir lieu d'informations, des informations de corrélation servant à corréler l'un ou l'autre parmi chaque partie traitée et chaque article absorbant ayant cette partie traitée avec la section de réalisation de traitement qui a formé cette partie traitée parmi la pluralité de sections de réalisation de traitement,
dans lequel
en laissant les parties traitées être des premières parties traitées, le corps rotatif être un premier corps rotatif, la position prédéterminée être une première position prédéterminée, et les sections de réalisation de traitement être des premières sections de réalisation de traitement,
les premières sections de réalisation de traitement sont mises en œuvre au niveau d'un premier intervalle angulaire dans la direction de rotation,
le procédé comporte par ailleurs une deuxième étape de formation de partie traitée consistant à former une deuxième partie traitée dans chaque première partie traitée,
la deuxième étape de formation de partie traitée est effectuée au niveau d'une deuxième position prédéterminée dans la direction de transport qui est différente de la première position prédéterminée,
la deuxième étape de formation de partie traitée comprend l'étape consistant à former les deuxièmes parties traitées par plusieurs deuxièmes sections de réalisation de traitement,
lesdites plusieurs deuxièmes sections de réalisation de traitement sont mises en œuvre selon un deuxième intervalle angulaire dans une direction de rotation sur une surface circonférentielle extérieure d'un deuxième corps rotatif,
le deuxième corps rotatif étant agencé au niveau de la deuxième position prédéterminée et tournant le long de la direction de transport,
les informations de corrélation sont des informations servant à corréler une première section de réalisation de traitement avec une deuxième section de réalisation de traitement,
la première section de réalisation de traitement étant l'une de la pluralité de premières sections de réalisation de traitement et formant une certaine première partie traitée,
la deuxième section de réalisation de traitement étant l'une desdites plusieurs deuxièmes sections de réalisation de traitement et formant la deuxième partie traitée dans la certaine première partie traitée,
le nombre de premières sections de réalisation de traitement mises en œuvre sur le premier corps rotatif est différent du nombre de deuxièmes sections de réalisation de traitement mises en œuvre sur le deuxième corps rotatif,
l'un ou l'autre parmi le nombre de premières sections de réalisation de traitement mises en œuvre sur le premier corps rotatif et le nombre de deuxièmes sections de réalisation de traitement mises en œuvre sur le deuxième corps rotatif est un nombre premier, et
ledit un autre parmi les nombres n'est pas un multiple entier (à l'exclusion de 0 et de 1) du nombre premier.

3. Procédé de fabrication d'un article absorbant selon la revendication 1, dans lequel
parmi les articles absorbants, un article absorbant déterminé comme étant un produit défectueux est déchargé au niveau d'une position en aval dans la direction de transport par rapport à la troisième position prédéterminée.

4. Procédé de fabrication d'un article absorbant selon l'une quelconque des revendications 1 à 3, dans lequel
le procédé comporte par ailleurs :
une étape consistant à placer une pluralité des articles absorbants dans un corps formant sac d'emballage ; et
une deuxième étape de génération d'informations servant à générer des deuxièmes informations,
les deuxièmes informations étant des informations servant à corréler la pluralité d'articles absorbants avec le corps formant sac d'emballage dans lequel les articles absorbants ont été placés.

5. Dispositif de fabrication d'un article absorbant, comportant :
un dispositif de transport configuré pour transporter une feuille continue dans une direction de transport,
la feuille continue étant continue dans la direction de transport ;
un dispositif de formation de partie traitée configuré pour former de manière répétée une partie traitée dans une partie de la feuille continue dans la direction de transport,
la partie étant une partie d'unité qui est destinée à devenir l'article absorbant,
l'étape de formation étant effectuée au niveau d'une position prédéterminée dans la direction de transport,
le dispositif de formation de partie traitée formant les parties traitées par une pluralité de sections de réalisation de traitement,
les sections de la pluralité de sections de réalisation de traitement étant mises en œuvre au niveau de différentes positions dans une direction de rotation sur une surface circonférentielle extérieure d'un corps rotatif,
le corps rotatif étant agencé au niveau de la position prédéterminée dans la direction de transport et tournant le long de la direction de transport ; et
un dispositif de génération d'informations configuré pour générer des informations se rapportant à l'un ou l'autre parmi chaque partie traitée et l'article absorbant qui a cette partie traitée,
le dispositif de génération d'informations générant, pour tenir lieu d'informations, des informations de corrélation servant à corréler l'un ou l'autre parmi chaque partie traitée et chaque article absorbant ayant cette partie traitée avec la section de réalisation de traitement qui a formé cette partie traitée parmi la pluralité de sections de réalisation de traitement,
dans lequel
la rotation du corps rotatif et le transport de la feuille continue sont coordonnés l'une par rapport à l'autre en fonction d'un signal de synchronisation constitué par un signal d'unité qui est émis en sortie de manière répétée,
à chaque fois que le signal d'unité est émis en sortie, uniquement l'une des parties qui sont destinées à devenir les articles absorbants dans la feuille continue est transportée dans la direction de transport,
un identifiant de section de réalisation de traitement est réglé de manière non redondante pour chaque section de réalisation de traitement,
à chaque fois que le signal d'unité est émis en sortie,
le corps rotatif tourne de telle sorte qu'une partie traitée est formée par l'une des sections de réalisation de traitement, et
des informations indiquant l'identifiant de section de réalisation de traitement de ladite une section de réalisation de traitement qui a formé cette partie traitée sont émises en sortie pour tenir lieu d'informations de corrélation,
au niveau d'une troisième position prédéterminée dans la direction de transport, à chaque fois que le signal d'unité est émis en sortie,
l'article absorbant ou une partie destinée à devenir l'article absorbant passe par la troisième position prédéterminée,
un identifiant est fourni de manière non redondante sur l'article absorbant ou la partie destinée à devenir l'article absorbant qui passe, et
des informations indiquant l'identifiant sont émises en sortie pour tenir lieu d'informations de corrélation, et
les informations indiquant l'identifiant et les informations indiquant l'identifiant de section de réalisation de traitement sont corrélées les unes avec les autres en fonction d'une somme d'un nombre d'articles absorbants et d'un nombre de parties destinées à devenir l'article absorbant,
les articles absorbants et les parties étant ceux qui existent entre la position prédéterminée et la troisième position prédéterminée.

6. Dispositif de fabrication d'un article absorbant, comportant :
un dispositif de transport configuré pour transporter une feuille continue dans une direction de transport,
la feuille continue étant continue dans la direction de transport ;
un dispositif de formation de partie traitée configuré pour former de manière répétée une partie traitée dans une partie de la feuille continue dans la direction de transport,
la partie étant une partie d'unité qui est destinée à devenir l'article absorbant,
l'étape de formation étant effectuée au niveau d'une position prédéterminée dans la direction de transport,
le dispositif de formation de partie traitée formant les parties traitées par une pluralité de sections de réalisation de traitement,
les sections de la pluralité de sections de réalisation de traitement étant mises en œuvre au niveau de différentes positions dans une direction de rotation sur une surface circonférentielle extérieure d'un corps rotatif,
le corps rotatif étant agencé au niveau de la position prédéterminée dans la direction de transport et tournant le long de la direction de transport ; et
un dispositif de génération d'informations configuré pour générer des informations se rapportant à l'un ou l'autre parmi chaque partie traitée et l'article absorbant qui a cette partie traitée,
le dispositif de génération d'informations générant, pour tenir lieu d'informations, des informations de corrélation servant à corréler l'un ou l'autre parmi chaque partie traitée et chaque article absorbant ayant cette partie traitée avec la section de réalisation de traitement qui a formé cette partie traitée parmi la pluralité de sections de réalisation de traitement,
dans lequel
en laissant les parties traitées être des premières parties traitées, le corps rotatif être un premier corps rotatif, la position prédéterminée être une première position prédéterminée, et les sections de réalisation de traitement être des premières sections de réalisation de traitement,
les premières sections de réalisation de traitement sont mises en œuvre au niveau d'un premier intervalle angulaire dans la direction de rotation,
le procédé comporte par ailleurs une deuxième étape de formation de partie traitée consistant à former une deuxième partie traitée dans chaque première partie traitée,
la deuxième étape de formation de partie traitée est effectuée au niveau d'une deuxième position prédéterminée dans la direction de transport qui est différente de la première position prédéterminée,
la deuxième étape de formation de partie traitée comprend l'étape consistant à former les deuxièmes parties traitées par plusieurs deuxièmes sections de réalisation de traitement,
lesdites plusieurs deuxièmes sections de réalisation de traitement sont mises en œuvre selon un deuxième intervalle angulaire dans une direction de rotation sur une surface circonférentielle extérieure d'un deuxième corps rotatif,
le deuxième corps rotatif étant agencé au niveau de la deuxième position prédéterminée et tournant le long de la direction de transport,
les informations de corrélation sont des informations servant à corréler une première section de réalisation de traitement avec une deuxième section de réalisation de traitement,
la première section de réalisation de traitement étant l'une de la pluralité de premières sections de réalisation de traitement et formant une certaine première partie traitée,
la deuxième section de réalisation de traitement étant l'une desdites plusieurs deuxièmes sections de réalisation de traitement et formant la deuxième partie traitée dans la certaine première partie traitée,
le nombre de premières sections de réalisation de traitement mises en œuvre sur le premier corps rotatif est différent du nombre de deuxièmes sections de réalisation de traitement mises en œuvre sur le deuxième corps rotatif,
l'un ou l'autre parmi le nombre de premières sections de réalisation de traitement mises en œuvre sur le premier corps rotatif et le nombre de deuxièmes sections de réalisation de traitement mises en œuvre sur le deuxième corps rotatif est un nombre premier, et
ledit un autre parmi les nombres n'est pas un multiple entier (à l'exclusion de 0 et de 1) du nombre premier.

7. Dispositif de fabrication d'un article absorbant selon la revendication 5, dans lequel
parmi les articles absorbants, un article absorbant déterminé comme étant un produit défectueux est déchargé au niveau d'une position en aval dans la direction de transport par rapport à la troisième position prédéterminée.
